# EUROPÄISCHE PATENTSCHRIFT

(11) **EP 1 401 425 B1**
(45) Veröffentlichungstag und Bekanntmachung des Hinweises auf die Patenterteilung: **16.08.2006**
(21) Anmeldenummer: 02760216.8
(22) Anmeldetag: 02.07.2002
(51) Int. Cl.: A61K 31/28, A61K 31/30, A61K 31/315, A61K 31/295, C07F 1/08, C07F 3/06, C07F 15/04, C07F 15/06, C07F 15/02, C07F 13/00, C07F 11/00

(54) **METALLORGANISCHES ANTITUMORMITTEL**
ORGANOMETALLIC ANTI-TUMOUR AGENT
AGENT ANTITUMORAL ORGANOMETALLIQUE

(30) Priorität: 03.07.2001 UZ 0100552; 17.01.2002 DE 10201693
(43) Veröffentlichungstag der Anmeldung: 31.03.2004
(73) Patentinhaber: Tatarsky, Valeriy, Prof. Dr., 14469 Potsdam (DE)
(72) Erfinder: Tatarsky, Valeriy, Prof. Dr., 14469 Potsdam (DE)
(74) Vertreter: Ziebig, Marlene
(86) Internationale Anmeldenummer: PCT/EP2002/007299
(87) Internationale Veröffentlichungsnummer: WO 2003/004014

(56) Entgegenhaltungen:
- WO-A-90/06749
- DD-A- 121 525
- US-A- 4 814 391
- US-A- 5 225 561
- US-A- 6 110 529
- TEMBE G L ET AL: "Manganese(II) beta-diketonate catalysed oxidation of cyclohexane by tert-butyl hydroperoxide" REACTION KINETICS AND CATALYSIS LETTERS, ELSEVIER, AMSTERDAM, NL, Bd. 63, Nr. 2, 1998, Seiten 385-389, XP001106905 ISSN: 0133-1736
- NATARAJAN C ET AL: "Synthesis, spectral studies and reactivity of nickel(II), copper(II) and zinc(II) mixed ligand complexes with 2-formyl, 2-acetyl and 2-benzoyl- cyclohexanones and acetylacetone" SYNTHESIS AND REACTIVITY IN INORGANIC AND METALLORGANIC CHEMISTRY, MARCEL DEKKER INCORPORATED, NEW YORK, US, Bd. 22, Nr. 6, 1992, Seiten 827-849, XP001106127 ISSN: 0094-5714
- BENNETT, ALISON M. A. ET AL: "The IR and proton, carbon-13 NMR spectra of the nickel(II), copper (II) and zinc(II) complexes of 2,4-pentanedione, 4-imino-2-pentanone, 4-thioxo-2-pentanone and 2,4-pentanedithione: a comparative study" POLYHEDRON, PERGAMON PRESS, OXFORD, GB, Bd. 8, Nr. 18, 1989, Seiten 2305-2311, XP001106503 ISSN: 0277-5387
- SUCH K P ET AL: "E.P.R. of Mn(2+) in acetylacetonates" MOLECULAR PHYSICS, LONDON, GB, Bd. 60, Nr. 3, 1987, Seiten 553-560, XP001106702
- KIRSTE, BURKHARD ET AL: "ENDOR study of bis(acetylacetonato) copper (II) in solid solution" JOURNAL OF PHYSICAL CHEMISTRY, AMERICAN CHEMICAL SOCIETY, US, Bd. 87, Nr. 5, 3. März 1983 (1983-03-03), Seiten 781-788, XP001105567 ISSN: 0022-3654
- DATABASE CA [Online] CHEMICAL ABSTRACTS SERVICE, COLUMBUS, OHIO, US; BOGUSLAVSKII, E. G. ET AL: "ESR study of copper (II) complexes with substituted thioureas" retrieved from STN Database accession no. 94:40629 XP002218071 -& IZVESTIJA SIBIRSKOGO OTDELENIJA AKADEMII NAUK SSSR, SERIJA CHIMICESKICH NAUK, Nr. 5, 1980, Seiten 50-53, XP001106290 ISSN: 0002-3426
- PETTY, RANDALL H. ET AL: "Cytochrome oxidase models. 2. .mu.-Bipyrimidyl mixed-metal complexes as synthetic models for the iron/ copper binuclear active site of cytochrome oxidase" JOURNAL OF THE AMERICAN CHEMICAL SOCIETY, AMERICAN CHEMICAL SOCIETY, US, Bd. 102, Nr. 2, 16. Januar 1980 (1980-01-16), Seiten 611-620, XP002218070 ISSN: 0002-7863
- MAROV, I. N. ET AL: "EPR study of the formation of adducts of copper (II) chelates with organic bases" RUSSIAN JOURNAL OF INORGANIC CHEMISTRY, CONSULTANTS BUREAU, US, Bd. 23, Nr. 10, 1978, Seiten 1495-1501, XP001106118 ISSN: 1070-4280
- HAIDER, S. Z. ET AL: "Copper complexes of the derivatives of 2,4-pentanedione" JOURNAL OF THE BANGLADESH ACADEMY OF SCIENCES, Bd. 1, Nr. 2, 1977, Seiten 53-62, XP001105570 ISSN: 0378-8121
- KWIATKOWSKI, EDMUND: "Calculation of formation constants for 1:1 complexes from spectrophotometric data" JOURNAL OF INORGANIC AND NUCLEAR CHEMISTRY, PERGAMON PRESS LIMITED, OXFORD, GB, Bd. 39, Nr. 9, 1977, Seiten 1611-1613, XP001106399
- LODZINSKA, ALICJA ET AL: "X-ray and luminescence spectral investigation of chelates of zinc(II) and some other transition metals with.beta.-diketones of the R-CO-CH2-CO-R' type" ROCZNIKI CHEMII, ANNALES SOCIETATIS CHEMICAE POLONORUM, Bd. 51, Nr. 3, 1977, Seiten 425-432, XP001117460
- TAMURA, HIROSHI ET AL: "DNA binding and cleaving activity of antitumor metal complex: sodium trans-dinitrobis(2,4-pentanedionato)cobalt (III)" CHEMISTRY LETTERS, CHEMICAL SOCIETY OF JAPAN, TOKYO, JP, Nr. 8, 1997, Seiten 711-712, XP001106300

## Beschreibung

Die vorliegende Erfindung betrifft die Verwendung einer pharmazeutischen Zusammensetzung enthaltend mindestens einen Komplex der allgemeinen Formel

D₂-M-T,

wobei
D ein β-Diketon, M ein Metallatom, ausgewählt aus der Gruppe, bestehend aus Cr, Cu, Mn, Fe, Ni, Co, Zn und Mo darstellt, und T eine Substanz mit mindestens einer N-, 0- oder S-enthaltenden Gruppe darstellt, wobei M mit T in einer Elektronen-Donor-Akzeptor-Wechselwirkung steht und M im Komplex eine freie Koordinationsstelle aufweist, zur Herstellung eines Antitumormittels.

In Deutschland erkranken jährlich 300.000 Menschen an Krebs und etwa jeder fünfte Deutsche stirbt an einer Tumorerkrankung, eine Zahl, die zweifelsohne in den nächsten Jahren zunehmen wird. Etwa 55 % aller Krebspatienten werden mit einer noch örtlich begrenzten Tumorerkrankung diagnostiziert, während bei den restlichen 45 % eine bereits fortgeschrittene, metastasierende Tumorerkrankung vorliegt. Viele Krebserkrankungen können jedoch durch rechtzeitige Diagnostik und Therapie geheilt werden. Grundsätzlich gibt es verschiedene Therapieformen, um Krebs zu behandeln. Hauptziel einer jeden Krebstherapie ist dabei jedoch immer die maximale Zerstörung und Entfernung aller Tumorzellen bei möglichst geringer Schädigung des den Tumor umgebenden normalen Gewebes.

Örtlich begrenzte Tumorerkrankungen werden vorwiegend durch lokale Therapiemaßnahmen, wie Chirurgie und Strahlentherapie, behandelt. Bei der Chirurgie wird der Primärtumor durch eine Operation möglichst vollständig entfemt, während die Tumorzellen im Primärtumor mit Hilfe der Strahlentherapie durch gezielte Bestrahlung abgetötet werden. Angriffsort der Bestrahlung ist die im Zellkern jeder Zelle befindliche DNA. Die Bestrahlung führt zu einer Vielzahl von DNA-Schäden, die nicht mehr vollständig von zelleigenen Enzymen repariert werden können. Als Folge davon leitet die Zelle den programmiert durchgeführten Zelltod ein. In weiteren Schritten werden die geschädigten Zellen aufgelöst und die dabei entstehenden Fragmente werden vom Immunsystem des Körpers abgebaut.

Örtlich begrenzte Tumorerkrankungen können sich jedoch über die Lymph- und Blutbahnen ausbreiten. Haben sich schon Metastasen in anderen Organen des Körpers angesiedelt, so reichen die lokalen Therapiemaßnahmen alleine nicht mehr aus, um die weitere Ausbreitung der Tumorerkrankung zu stoppen. In diesen Fällen muss die Behandlung den gesamten Körper umfassen, was durch eine Chemotherapie erreicht werden kann. Bei der Chemotherapie werden gezielt Substanzen, nämlich Zytostatika, verabreicht, welche das Wachstum von Tumorzellen hemmen und somit die Tumorzellen abtöten. Zu den bekannten Zytostatika zählen Antimetabolite, Topoisomerase-Hemmer, alkylierende Wirkstoffe und pflanzliche Alkaloide. Obwohl alle Zytostatika eine Hemmung des Tumorzellwachstums bewirken, sind die den verschiedenen Zytostatika zugrundeliegenden. Wirkprinzipien völlig verschieden. Ein möglichst großes Spektrum von Zytostatika mit unterschiedlichen Wirkprinzipien ist für die Behandlung der verschiedenen Tumorerkrankungen von entscheidender Bedeutung, da jede Tumorerkrankung anders ist und eine spezifische Art der Behandlung benötigt. Trotz der großen Anzahl der bislang bekannten Zytostatika können bislang noch nicht alle Tumorerkrankungen therapiert werden. Daher besteht immer noch ein fortwährendes Interesse an der Entwicklung neuartiger Zytostatika.

Aus DD-WP 121 525 ist die Verwendung stickstoffhaltiger Metallchelate als Prooxidantien für oxydativ trocknende Überzugsmassen bekannt. Sie werden vorwiegend als Anstrichstoffe und Beschichtungsmittel eingesetzt.

Von Tamura, Hiroshi et al (Chemistry Letters, Chemical Society of Japan, Tokyo, Japan, Nr. 8, 1997, Seiten 711-712) werden der Metallkomplex Natrium trans-Dinitrobis(2,4-pentandionat)kobalt(III) als Antitumor-Metallkomplex beschrieben. Dieser Kobaltkomplex ist ein Elektrolyt, der aus einem Natrium-Kation und dem komplexen Anion besteht, weiches durch Resonanzwechselwirkung zwischen den Elektronen von NO₂-Gruppen und Acetylacetonatgruppen stabilisiert ist. Die Wechselwirkung mit biologischen Substanzen, z.B. mit DNA bedarf einer erheblichen Aktivierung, z.B. durch Licht. Eine selektive Wirkung auf Tumorzellen ist nicht dargestellt.

Der vorliegenden Erfindung lag die Aufgabe zugrunde, Metallkomplexe für die Tumorbekämpfung zu finden und mit diesen Zytostatika mit hoher Antitumorwirksamkeit und breitem Wirkungsspektrum gegenüber einer Vielzahl von Tumorerkrankungen herzustellen.

Diese Aufgabe wurde erfindungsgemäß durch Verwendung einer pharmazeutischen Zusammensetzung enthaltend mindestens einen Komplex der allgemeinen Formel

D₂-M-T

zur Herstellung eines Antitumormittels gelöst, wobei
- D: ein β-Diketon darstellt,
- M: ein Metallatom, ausgewählt aus der Gruppe, bestehend aus Cr, Cu, Mn, Fe, Ni, Co, Zn und Mo darstellt,
- T: eine Substanz mit mindestens einer N-, 0- oder S-enthaltenden Gruppe darstellt und
wobei M mit T in einer Elektronen-Donor-Akzeptor-Wechselwirkung steht und M im Komplex eine freie Koordinationsstelle aufweist.

Die monokristallinen, metallorganischen Komplexverbindungen besitzen eine tetragonal-bipyramidale Geometrie. Das Metallatom M des Komplexes befindet sich im Zentrum der tetragonalen Bipyramide. Die beiden zweizähnigen β-Diketonliganden D nehmen jeweils mit ihren beiden Komplexbildenden Sauerstoffatomen die vier äquatorialen Positionen der tetragonalen Bipyramide ein. Eine der beiden axialen Positionen der tetragonalen Bipyramide ist durch die Substanz T besetzt, wobei das N-bzw. 0- bzw. S-Atom der N- bzw. 0- bzw. S-enthaltenden Gruppe der Substanz T als Komplex-bildendes Atom dient und in einer Elektronen-Donor-Akzeptor-Wechselwirkung mit dem Metallatom M steht. An der anderen axialen Position der tetragonalen Bipyramide befindet sich eine freie Koordinationsstelle. Die freie Koordinationsstelle am Metallatom M des erfindungsgemäßen Komplexes ermöglicht eine spezifische Wechselwirkung mit anderen Molekülen, wie z.B. mit Sauerstoff, Stickoxiden oder einer molekularen Bindungsstelle auf der Oberfläche von Target-Zellen etc.

Strukturelle Untersuchungen konnten zeigen, dass das Metallatom M des Komplexes etwa 0,2 Å von seiner idealen Position in der tetragonalen Bipyramide in Richtung der Substanz T abweicht. Dadurch nimmt die Elektronen-Donor-Akzeptor-Wechselwirkung zwischen dem Metallatom M und der Substanz T den Charakter einer Doppelbindung an. Des Weiteren konnte gezeigt werden, dass zwei der vier äquatorialen Positionen etwas in Richtung des Metallatoms verlagert sind, während die anderen beiden äquatorialen Positionen etwas von dem Metallatom entfemt angeordnet sind.

Das β-Diketon D des Komplexes ist dadurch ausgezeichnet, dass seine dreidimensionale Struktur (i) eine optimale Chelatbildung mit dem Metallatom M an dessen äquatorialen Koordinationsstellen ermöglicht und (ii) die Elektronen-Donor-Akzeptor-Wechselwirkung zwischen der Substanz T und dem Metallatom M nicht stört. Vorzugsweise ist das β-Diketon jedoch ausgewählt aus der Gruppe, bestehend aus Acetylaceton und dessen höheren Alkylanalogen und Dibenzoylmethan.

Das Metallatom M des Komplexes ist ausgewählt aus der Gruppe, bestehend aus Cr, Cu, Mn, Fe, Ni, Co, Zn und Mo. Überdies ist das Metallatom M dadurch gekennzeichnet, dass es eine tetragonal-bipyramidale Anordnung der Liganden D und T ermöglicht, wobei eine axiale Koordinationsstelle des Metallatoms frei bleibt. Besonders bevorzugte Metallatome M sind Cu und Mn.

Die Substanz T des Komplexes weist mindestens eine N-, O- oder S-enthaltende Gruppe auf, die über das N- bzw. O- bzw. S-Atom eine Elektronen-Donor-Akzeptor-Wechselwirkung mit dem Metallatom M an einer der axialen Koordinationsstellen von M eingeht. Dabei wirkt das N- bzw. 0- bzw. S-Atom der Substanz T als Elektronendonor und stellt dem Metallatom M als Elektronenakzeptor ein freies Elektronenpaar zur Verfügung. Vorzugsweise weist die Substanz T mindestens eine NH₂-, NH-, N-, O- oder S-enthaltende Gruppe auf. In einer bevorzugten Ausführungsform der Erfindung weist die Substanz T bereits eine Antitumorwirksamkeit auf und ist ausgewählt aus der Gruppe, bestehend aus 2,4-Dihydroxy-5-fluorpyrimidin, 5-Fluor-1-(tetrahydro-2-furyl)-uracil, 2-[Bis-(2-chlorethyl)-amino]-tetrahydro-2H-1,3,2-oxazephosphorin-2-oxid, 1,2-Imidopropylsäureamid (Leacadin), 2-Hydroxymethyl-5-hydroxy-γ-pyrone, 2,4,6-Trimethylpyridin, 2,4,6-Tri-2-pyridyl-1,3,5-triazin, 4-[Bis-(2-chlorethyl)-amino]-L-phenylalanin (Melphalan), 2-(3-Pyridyl)-piperidin, 2-2'-Bipyridin, 2-Methyl-(5-trimethyl-butyl-1-il-ol-3)-pyridin, 2-Methyl-(3-dimethyl-amino-1-propinyl)-pyridin und 2-Methyl-5-ethylen-pyridin.

In einer bevorzugten Ausführungsform der Erfindung umfasst der Komplex Kupfer als zentrales Metallatom M, Acetylaceton oder ein höheres Alkylanaloges desselben als β-Diketon D und eine Substanz, ausgewählt aus der Gruppe, bestehend aus 2,4-Dihydroxy-5-fluorpyrimidin, 5-Fluor-1-(tetrahydro-2-furyl)-uracil, 2-[Bis-(2-chlorethyl)-amino]-tetrahydro-2H-1,3,2-oxazephosphorin-2-oxid, 1,2-lmidopropylsäureamid, 2-Hydroxymethyl-5-hydroxy-γ-pyrone, 2,4,6-Trimethylpyridin, 2,4,6-Tri-2-pyridyl-1,3,5-triazin, 4-[Bis-(2-chlorethyl)-amino]-L-phenylalanin, 2-(3-Pyridyl)-piperidin, 2-2'-Bipyridin, 2-Methyl-(5-trimethyl-butyl-1-il-ol-3)-pyridin, 2-Methyl-(3-dimethyl-amino-1-propinyl)-pyridin und 2-Methyl-5-ethylen-pyridin als Substanz T.

In einer weiteren bevorzugten Ausführungsform der Erfindung umfasst der Komplex Mn als zentrales Metallatom M, Acetylaceton oder dessen höhere Alkylanaloga als β-Diketon D und eine Substanz, ausgewählt aus der Gruppe, bestehend aus 2,4,6-Trimethylpyridin, 2,4,6-Tri-2-pyridyl-1,3,5-triazin, 2-2'-Bipyridin, 2-(3-Pyridyl)-piperidin, 1,2-Imidopropylsäureamid und 4-[Bis-(2-chlorethyl)-amino]-L-phenylalanin als Substanz T.

Es hat sich nun überraschenderweise gezeigt, dass der Komplex zur Herstellung von Zytostatika mit ausgezeichneter Antitumorwirksamkeit geeignet ist. Stellt die Substanz T in einer bevorzugten Ausführungsform des Komplexes bereits ein Antitumormittel dar, wurde festgestellt, dass der Komplex eine im Vergleich zu der darin enthaltenen Substanz T stark erhöhte Antitumorwirksamkeit zeigt. Zudem weist der Komplex immunmodulatorische und antiproliferative Eigenschaften sowie eine anti-angiogene Wirksamkeit auf und besitzt eine gegenüber herkömmlichen Antitumormitteln stark erhöhte Hydrolysestabilität, wodurch er in weiten Bereichen der Tumorbekämpfung einsetzbar ist. Des Weiteren konnte festgestellt werden, dass der Einsatz des Komplexes keine Arzneimittelresistenz hervorruft und unter bestimmten Bedingungen in der Lage ist, Apoptose und Angiogenese bei Krebszellen herbeizuführen.

Besonders bevorzugt wird im Rahmen der Erfindung der Komplex aus Kupfer oder Mangan, Acetylaceton und 4-[Bis-(2-chlorethyl)-amino]-L-Phenylalanin (Melphalan), im Folgenden als MOC-Melphalan bezeichnet, verwendet. Die durchgeführten Untersuchungen zeigen, dass sich diese Substanz überwiegend im Tumorgewebe anreichert, katalytisch Fragmente von Proteinrezeptoren auf der Membranoberfläche oxidiert und damit Metastasierungsprozessen vorbeugt. Die verwendete Substanz verfügt weiter über immunmodulierende Wirkungen über die Regulation des Verhältnisses Tₕₑₗₚ/Tₛᵤₚᵣ und beeinflusst die Bildung von spezifischen Antikörpern. Die Substanz ist chemisch stabil und hat eine lang anhaltende Wirkung. Von besonderer Bedeutung ist, dass die Substanz die Blut-Hirn-Schranke überwindet und damit die Behandlung von Gehimtumoren ermöglicht. Außerdem wird keine Arzneimittelresistenz erzeugt. Untersuchungen am Adenokarzinom, Sarkom, Leukämiezellen, Melanom und Nierenzellkarzinom (RENCA) ergaben eine im Vergleich zu Melphalan eine weit überlegene Wirksamkeit.

Hinsichtlich des Wirkungsmechanismus dieser besonders bevorzugten Substanz wird angenommen, dass sie den Stickstoffmonoxidgehalt und die Ca-Zonenkonzentrationen in den Tumorzellen zu regulieren vermag. Durch "Absaugung" der Ca²⁺-lonen aus den Tumorzellen wird der Glykolyseprozess inhaliert und damit die Funktion ihrer Mitochondrien beeinträchtigt. Bei der Rekombination der aktiven Formen des Sauerstoffs entstehen durch MOC-Melphalan Bedingungen, die zur Zerstörung der Tumorzellen unter Mithilfe der Makrophagen - die mit NO beladen sind - führen. Außerdem wirkt die Substanz auf die Genexpression durch Eindringen in den Kern der Tumorzellen, zerstört dadurch den Zellkem und inhaliert die Proliferationsaktivität. Schließlich wurde festgestellt, dass sie auch eine zweischichtige Kapselbildung des Tumors hervorruft, wobei die innere Schicht aus fettähnlichen Zellen besteht. Hierdurch wird die Nährstoffversorgung des Tumors zusätzlich unterdrückt und gegebenenfalls ein gezielter chirurgischer Eingriff ermöglicht.

Eine weitere besonders bevorzugte Substanz aus den erfindungsgemäß verwendeten Komplexen ist der Komplex von Kupfer-Acetylacetonat mit Tegafur:
Cu(C₅H₇0₂)_{2•}C₈H₉O₃N₂F - MOC-Tegafur.

Die entscheidenden Nachteile von Tegafur [5-Fluoro-1-(tetrahydro-2-furyl)-uracil sind die kurze Wirkdauer, die nur zu einer Unterdrückung der Synthese von Nukleinsäuren führt, und die schlechte Löslichkeit in Wasser. Trotz seiner niedrigen Toxizität (LD₅₀ beträgt 650 mg/kg) zeigt das Präparat eine starke Wirkung auf die Blutbildung und ruft Leukopenie, Thrombozytopenie und Anemie hervor. Es reichert sich in hohen Konzentrationen im Gehirngewebe an und ruft Diarrhoe und Stomatitis hervor. Das Präparat Tegafur darf nicht bei Nieren- und Leber-Krankheiten (im terminalen Zustand), bei Blutungen und bei einem Gehalt an Leukozyten- und Thrombozyten unter 3·10⁹/l angewendet werden. Die Anwendung des Tegafurs ist begrenzt bei der Behandlung von Dünn- und Dickdarmtumoren, rezidiven Magentumoren, sowie bei Mamma- und Eierstockkarzinom.

Die Kombination des Moleküls Tegafur mit dem Kupfer-Acetylacetonat-Molekül führt zu einer chemischen Verbindung, die diese zahlreichen Nachteile nicht aufweist. Der metallorganische Komplex MOC·Tegafur verfügt über ein breites Spektrum an antitumorwirkenden, antimetastasierenden und immunregulierenden Eigenschaften. Es ist eine wasserlösliche Substanz mit prolongierender Wirkung, und es ruft keine Arzneiresistenz im Organismus hervor. Ein weiterer entscheidender Vorteil ist die therapeutische Dosierung des Präparates MOC-Tegafur (Dosierung von 5 mg/kg Körpergewicht). Bei Tegafur allein beträgt diese nur 2,1 mg. Auch MOC-Leacadin (Cu/Mn(acac)₂-Leacadin) zeigt die bei MOC-Melphalan näher beschriebenen Wirkungen wie "Absaugung" der Ca²⁺-lonen, Zerstörung der Mitochondrien in Tumorzellen usw.

Die vorliegende Erfindung betrifft demzufolge die Verwendung einer pharmazeutischen Zusammensetzung, umfassend wenigstens einen Komplex zur Herstellung eines Antitumormittels. Die pharmazeutische Zusammensetzung kann einen einzigen Komplex oder eine Kombination aus mehreren Komplexen als Wirkstoff enthalten. Des Weiteren kann die pharmazeutische Zusammensetzung gegebenenfalls dem Fachmann hinreichend bekannte, herkömmlich verwendete pharmazeutische Zusatzstoffe enthalten, wie z.B. physiologisch verträgliche Trägersubstanzen, Verdünnungsmittel und Hilfsmittel.

Die pharmazeutische Zusammensetzung kann in einer topisch, parenteral, intravenös, intramuskulär, subkutan oder transdermal verabreichbaren Form vorliegen und kann mit Hilfe von herkömmlichen, im Stand der Technik gut bekannten Verfahren hergestellt werden. Vorzugsweise wird die pharmazeutische Zusammensetzung in Form von Tabletten oder als intravenöse Injektion bzw. Infusion hergestellt.

Die pharmazeutische Zusammensetzung wird zur Behandlung von Tumoren eingesetzt. Die Bezeichnung "Tumor", wie hierin verwendet, umfasst jede örtliche Zunahme des Gewebevolumens sowie Zellen, bei denen die normale Wachstumsregulation nicht mehr greift und die ungeregelte Zellteilung stattfindet. Das heißt im weitesten Sinne jede lokalisierte Anschwellung durch Ödem, akute und chronische Entzündung, aneurysmatische Erweiterung und entzündlich bedingte Organschwellung, sowie im engsten Sinne eine gewebliche Neubildung (z.B. Gewächs, Blastom, Neoplasie) in Form eines spontanen, verschiedengradig enthemmten, autonomen und irreversiblen Überschusswachstums von körpereigenem Gewebe, das in der Regel mit unterschiedlich ausgeprägtem Verlust spezifischer Zell- und Gewebefunktionen verbunden ist. Beispiele für Tumorerkrankungen, die mit Hilfe der pharmazeutischen Zusammensetzung behandelt werden können, umfassen Darmkrebs, Himtumor, Augentumor, Pankreaskarzinom, Harnblasenkarzinom, Lungenkrebs, Brustkrebs, Ovarialtumor, Gebärmutterkrebs, Knochentumor, Gallenblasen- und Gallengangskarzinom, Kopf-Hals-Tumor, Hautkrebs, Hodenkrebs, Nierentumor, Keimzelltumor, Leberkrebs, Leukämie, malignes Lymphom, Nerventumor, Neuroplastom, Prostatakrebs, Weichteiltumor, Speiseröhrenkrebs sowie Karzinome bei unbekanntem Primärtumor.

Die Bezeichnung "Behandlung von Tumoren", wie hierin verwendet, umfasst wenigstens eines der folgenden Merkmale: eine Linderung der mit der Tumorerkrankung verbundenen Symptome, eine Verringerung des Ausmaßes der Tumorerkrankung (z.B. eine Reduktion des Tumorwachstums), eine Stabilisierung des Zustandes der Tumorerkrankung (z.B. eine Inhibierung des Tumorwachstums), eine Verhinderung einer weiteren Ausbreitung der Tumorerkrankung (z.B. eine Metastasierung), eine Verhinderung des Auftretens oder Wiederauftretens einer Tumorerkrankung, eine Verzögerung oder Verlangsamung des Verlaufs der Tumorerkrankung (z.B. eine Reduktion des Tumorwachstums) oder eine Verbesserung des Zustandes der Tumorerkrankung (z.B. eine Verkleinerung des Tumors).

Vorzugsweise wird die pharmazeutische Zusammensetzung einem Patienten mit einer Tumorerkrankung in einer Menge verabreicht, die ausreichend ist, um eine Behandlung des entsprechenden Tumors zu erzielen. Die zu verabreichende Menge der pharmazeutischen Zusammensetzung hängt dabei von mehreren Faktoren ab, wie z.B. der Wahl des Komplexes (Spezifität, Wirksamkeit etc.), der Art der Verabreichung (Tablette, Injektion, Infusion etc.), der Art und dem Ausmaß der Tumorerkrankung und dem Alter, Gewicht und Allgemeinzustand des Patienten, und kann ohne weiteres von einem Fachmann auf dem Gebiet der Tumorerkrankung unter Berücksichtigung der oben genannten Faktoren bestimmt werden. Vorzugsweise werden die Komplexe jedoch im Bereich von 1 µg/kg Körpergewicht des Patienten bis 5 mg/kg Körpergewicht des Patienten, vorzugsweise von 1 µg/kg Körpergewicht des Patienten bis 0,5 mg/kg Körpergewicht des Patienten und besonders bevorzugt von 10 µg/kg Körpergewicht des Patienten bis 0,1 mg/kg Körpergewicht des Patienten, verabreicht.

Die Verabreichung der pharmazeutischen Zusammensetzung erfolgt topisch, parenteral, intravenös, intramuskulär, subkutan oder transdermal. Vorzugsweise wird die pharmazeutische Zusammensetzung in Form von Tabletten oder als intravenöse Injektion bzw. Infusion verabreicht. In einzelnen Fällen kann auch eine gezielte Einspritzung der pharmazeutischen Zusammensetzung in Körperhöhlen oder über einen Katheter in die Blutgefäße der Tumorregion bzw. des Organs, in dem der Tumor sitzt, erfolgen.

Ein weiterer Gegenstand der vorliegenden Erfindung betrifft eine pharmazeutische Zusammensetzung der allgemeinen Formel

D₂ - M - T

worin D Acetylaceton, M Cu und T eine Substanz, ausgewählt aus der Gruppe, bestehend aus 2,4-Dihydroxy-5-fluorpyrimidin, 5-Fluor-1-(tetrahydro-2-furyl)-uracil (Tegafur), 2-[Bis-(2-chlorethyl)-amino]-tetrahydro-2H-1,3,2-oxazephosphorin-2-oxid, 1,2-Imidopropylsäureamid, 2-Hydroxymethyl-5-hydroxy-y-pyrone, 2,4,6-Trimethylpyridin, 2,4,6-Tri-2-pyridyl-1,3,5-triazin, 4-[Bis-(2-chlorethyl)-amino]-L-phenylalanin, 2-(3-Pyridyl)-piperidin, 2-2'-Bipyridin, 2-Methyl-(5-trimethyl-butyl-1-il-ol-3)-pyridin, 2-Methyl-(3-dimethylamino-1-propinyl)-pyridin und 2-Methyl-5-ethylen-pyridin, ist.

Des Weiteren betrifft sie eine pharmazeutische Zusammensetzung der allgemeinen Formel

D₂ - M - T

worin D Acetylaceton, M Mn und T eine Substanz, ausgewählt aus der Gruppe, bestehend aus 2,4,6-Trimethylpyridin, 2,4,6-Tri-2-pyridyl-1,3,5-triazin, 2-2'-Bipyridin, 2-(3-Pyridyl)-piperidin, 1,2-Imidopropylsäureamid und 4-[Bis-(2-chlorethyl)-amino]-L-phenylalanin, ist.

Die nachfolgenden Beispiele sollen die Erfindung näher erläutern in Verbindung mit den Abbildungen der beigefügten Zeichnung.

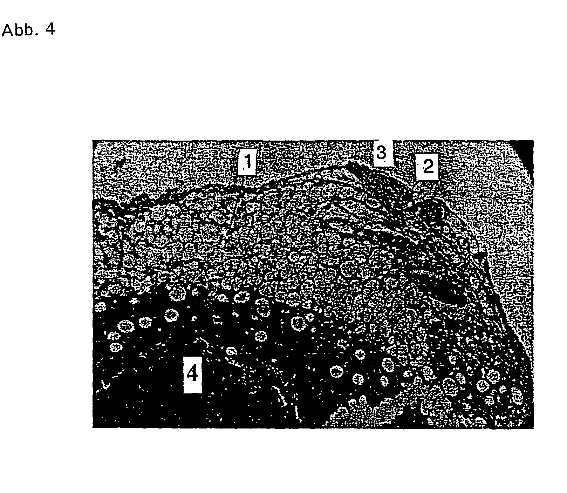

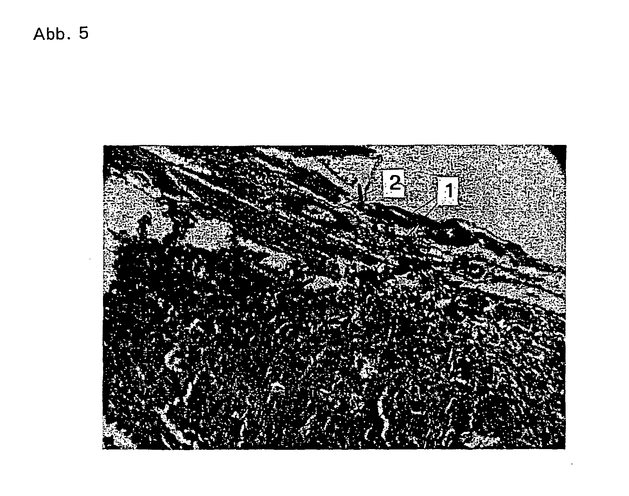

In den Zeichnungen stellen dar:
- Abb. 1:: Black-Linie-Maus mit Melanom B-16 nach Behandlung mit MOC·Melphalan, Tumorgewicht 0,3 g
- Abb. 2:: Black-Linie-Maus mit Melanom B-16, Kontrollgruppe, Tumorgewicht 3,15 g
- Abb.3:: DNS-Elektrophorese von Black-Linie-Mäusen mit Melanom B-16
2 MOC•Melphalan
4 Kontrollgruppe
1,3 Referenzsubstanzen
- Abb. 4:: Wirkung der Substanz MOC·Melphalan auf die Kapselbildung des Tumors Melanom 8-16 (100-fache Vergrößerung)
1 Parenchymfettzellen
2 epithelähnliche Zellen
3 Gefäß
4 Tumorgewebe
- Abb. 5:: Tumor Melanom B-16 in der Kontrollgruppe (100-fache Vergrößerung)
1 Muskelfaserpakete
2 Gefäß
- Abb. 6:: zeigt die präventive Wirkung der Substanz MOC·Melphalan auf den Tumor AKATON: Tumormasse und -volumen nach 4-maliger intravenöser Applikation von MOC·Melphalan vor Tumorimplantation
- Abb. 7:: zeigt ein Elektropherogramm der DNA von Zellen des Sarkom S-180 unter Einfluss der Substanz
MOC·Melphalan in einer Dosierung von 0,05 mg/10⁶ Zellen
2 MOC·Melphalan, Inkubationszeit 40 Minuten
5 MOC·Melphalan, Inkubationszeit 60 Minuten
7 Kontrollgruppe
1, 3, 4, 6 Referenzsubstanzen
- Abb. 8:: Bildung der doppelschichtigen Hülle zwischen bösartigem und gesundem Gewebe durch die Wirkung der Substanz MOC-Tegafur
- Abb. 9:: Bildung von Hohlräumen in den Zellen unter Einwirkung der Substanz MOC-Tegafur
- Abb. 10:: Wirkung der Substanz MOC-Tegafur auf die Kapselbildung des Tumors Melanom B-16 (Vergrößerung 40 × 40):
1 epithelähnliche Zellen mit Anzeichen von Degradation
2 Zellen der Muskelfasern
3 Tumorgewebe
- Abb. 11:: Wirkung der Substanz MOC Melphalan auf die Kapselbildung des Tumors Melanom B-16 (Vergrößerung: 10 × 10):
1 Parenchymfettzellen
2 epithelähnliche Zellen
3 Gefäß
4 Tumorgewebe
- Abb. 12:: Tumor Melanom B-16 bei Tieren der Kontrollgruppe (Vergrößerung 10 × 10)
1 Gewebebündel der Muskeln
2 Gefäße

### Beispiele

### Beispiel 1: Herstellung eines erfindungsgemäßen Komplexes am Beispiel von (C₅H₇O₂)₂-Cu-C₁₃H₁₈Cl₂N₂O₂

### a) Synthese von Cu(C₅H₇O₂)₂

Zu einer Lösung von 20,4 g CuCl × 2H₂O in 125 ml Wasser wurden 25 ml frisch destilliertes C₅H₉O₂, gelöst in 50 ml Methanol, unter ständigem Rühren gegeben. Dann wurde eine Lösung aus 20 g Natriumacetat in 75 ml Wasser zu dieser Mischung gegeben. Das so erhaltene Gemisch wurde im Wasserbad bis zum Sieden erhitzt und anschließend auf Zimmertemperatur abgekühlt. Das gebildete Cu(C₅H₇O₂)₂ wurde aus Methanol auskristallisiert. Einige Stunden nach der vollständigen Auskristallisation wurden die blauen Cu(C₅H₇O₂)₂-Kristalle abfiltriert, mit Wasser gewaschen und bei einer Temperatur von 80°C und einem Druck von 6 mm Hg vakuumgetrocknet.

### b) Synthese des Komplexes (C₅H₇O₂)₂-Cu-C₁₃H₁₈Cl₂N₂O₂

Zu 0,01 Mol Cu (C₅H₇O₂)₂ in 20 ml Lösungsmittel wurden unter ständigem Rühren 20 ml einer 0,02 molaren Lösung aus C₁₃H₁₈Cl₂N₂O₂ (4-[Bis(2-chlorethyl)-amino]-L-phenylalanin) gegeben.

Variante 1: Das Glasgefäß mit der so erhaltenen Lösung wurde mit einem Polyethylenverschluss abgedichtet und für einige Tage zur langsamen Auskristallisation an einem dunklen Ort gelagert. Nach einigen Tagen wurden die grünen (C₅H₇O₂)₂-Cu-C₁₃H₁₈Cl₂N₂O₂-Kristalle abgetrennt und mehrere Male mit einem Lösungsmittel von physikalisch anhaftenden C₁₃H₁₈Cl₂N₂O₂-Molekülen gereinigt. Danach wurden die (C₅H₇O₂)₂-Cu-C₁₃H₁₈Cl₂N₂O₂-Kristalle an der Luft getrocknet.

Variante 2: Die so erhaltene Lösung wurde in einem Rotationsverdampfer verdampft, wobei das Lösungsmittel unter Vakuumbedingungen (6 mm Hg) bei einer Temperatur von 40 °C abgesaugt wurde. Die grün gefärbten (C₅H₇O₂)₂-Cu-C₁₃H₁₈Cl₂N₂O₂-Kristalle wurden aus dem Glaskolben entnommen, mit Lösungsmittel gereinigt und an der Luft getrocknet.

### Beispiel 2: Hydrolysestabilität der erfindungsgemäßen Komplexe am Beispiel von (C₅H₇O₂)₂-Cu-C₁₃H₁₈Cl₂N₂O₂

### 1. Hydrolysestabilität in Wasser oder physiologischer Kochsalzlösung

0,6 g (C₅H₇O₂)₂-Cu-C₁₃H₁₈Cl₂N₂O₂ wurden mit Hilfe eines Ultraschallgenerators (Frequenz: 15 kH, 10 Minuten) in 100 ml Wasser oder physiologischer Kochsalzlösung dispergiert. Die so erhaltene Lösung war über einen Zeitraum von 30 Tagen bei 20°C stabil und zeigte in diesem Zeitraum keine Hydrolyse.

### II. Hydrolysestabilität in Olivenöl

0,6 g (C₅H₇O₂)₂-Cu-C₁₃H₁₈Cl₂N₂O₂ wurden mit Hilfe eines Ultraschallgenerators (Frequenz: 15 kH, 10 Minuten) in 100 ml 100 %-igem Olivenöl dispergiert. Die so erhaltene Lösung war über einen Zeitraum von mehr als 2 Jahren bei 20°C stabil und zeigte keine Hydrolyse.

### III. Hydrolysestabilität in Linolsäure oder Linolensäure

0,1 g (C₅H₇O₂)₂-Cu-C₁₃H₁₈Cl₂N₂O₂ wurden mit Hilfe eines Ultraschallgenerators (Frequenz: 15 kH, 10 Minuten) in 100 ml ,Linolsäure oder Linolensäure dispergiert. Die so erhaltene Lösung wies eine hellgrüne Farbe auf und war an der Luft bei 20°C über einen Zeitraum von 1 Jahr stabil.

### Beispiel 3: Antitumorwirksamkeit der erfindungsgemäßen Komplexe

1) 10 Mäusen der Linie Balb, welche ein Adenokarzinom enthielten, wurde Cu(acac)₂M intraabdominal in einer Dosis von 5 mg/kg in 0,3 ml physiologischer Kochsalzlösung verabfolgt. Zur Kontrolle wurden 10 weitere Mäuse der gleichen Art mit dem gleichen Tumor ohne Behandlung bestimmt. Bei den Kontrollmäusen wurde ein durchschnittliches Tumorgewicht von 3,60 g festgestellt, bei den behandelten Mäusen betrug das durchschnittliche Tumorgewicht 0,3 g. In der nachfolgenden Tabelle 1 sind die Ergebnisse bei den erfindungsgemäß behandelten Mäusen angegeben mit Gewicht und Größe des Tumors. Im Vergleich zur Kontrollserie ergibt sich daraus eine 91,9 %-ige Hemmung des Tumors.

**Tabelle 1**

| Mäuse | Gewicht des Tumors in g | Größe des Tumors cm |
|---|---|---|
| 1 | 0 | 0 |
| 2 | 0 | 0 |
| 3 | 0,35 | 0,2 × 0,2 × 0,2 |
| 4 | 1,13 | 1,4 × 0,7 × 0,6 |
| 5 | 0,8 | 0, 8 × 0, 8 × 0, 5 |
| 6 | 0 | 0 |
| 7 | 0 | 0 |
| 8 | 0 | 0 |
| 9 | 0,49 | 0,3×0,2×0,2 |
| 10 | verendet | - |

Die nachstehende Tabelle 2 zeigt die Ergebnisse der Kontrollserie unbehandelter Mäuse mit ebenfalls Gewicht und Größe des Tumors angegeben. Das durchschnittliche Gewicht des Tumors betrug 3,60 g.

**Tabelle 2**

| Mäuse | Gewichts des Tumors in g | Größe des Tumors cm |
|---|---|---|
| 1 | 1,61 | 1,6 × 1,0 × 0,7 |
| 2 | 1,6 9 | 2,5 × 2,0 × 0,8 |
| 3 | 6, 48 | 3,6 × 2,5 × 1,6 |
| 4 | 2,9 3 | 3,0 × 1,0 × 0,7 |
| 5 | 3,75 | 3,5 × 1,5 × 0,7 |
| 6 | 2,56 | 3,1 × 1,0 × 1,7 |
| 7 | 3,69 | 3,0 × 2,0 × 1,0 |
| 8 | 5,22 | 3,0 × 2,5 × 1,0 |
| 9 | 4,85 | 3,0 × 2,0 × 1,0 |
| 10 | 3,23 | 2,6 × 1,5 × 1,0 |

### 2. Antitumoraktivität bei Adenokarzinom und intravenöser Verabreichung (viermal in physiologischer Kochsalzlösung)

Die Ergebnisse zeigt die nachstehende Tabelle 3.

**Tabelle 3**

| Präparate | Dosis des Präparates | Anzahl der Tiere | Masse des Tumors g | % der Hemmung |
|---|---|---|---|---|
| (Cu(acaC)₂M | 5 mg/kg | 6 | 0,9 | 80 |
| Kontrolle | - | 6 | 4,4 | |

### 3. Antitumoraktivität bei Adenokarzinom, welcher nach Verabreichung des Präparates verpflanzt wurde.

**Tabelle 4**

| Präparate | Dosis des Präparates | Anzahl der Tiere | Masse des Tumors g | % der Hemmung | Ausmaß des Tumors in cm³ | % der Hemmung |
|---|---|---|---|---|---|---|
| Cu(acac)₂ M | 5 mg/kg | 6 | 0,91 | 80 | 1,38 | 82,5 |
| Kontrolle | | 6 | 4,46 | | 7,9 | |

Der erfindungsgemäße Wirkstoff wurde in physiologischer Kochsalzlösung von je 0,3 ml in der in der Tabelle angegebenen Dosierung 4 Tage hintereinander intravenös verabreicht. Danach wurde den Balb-Mäusen des Adenokarzinom eingepflanzt. 21 Tage nach Verpflanzung des Tumors wurden die Tiere getötet und Gewicht und Größe des Tumors registriert. Im Verlauf des Versuchs erhielten die Tiere keine sonstigen Arzneimittel und wurden bei normaler Futterration gehalten. Die Ergebnisse zeigen, dass der erfindungsgemäße Wirkstoff sich im Organismus anlagern kann und über eine prolongierende Wirkung verfügt.

### 4. Antitumoraktivität bei Sarkom C-180

In der nachstehenden Tabelle 5 sind die Ergebnisse von Versuchsreihen dargestellt, bei denen der angegebene Wirkstoff intraperitoneal in physiologischer Kochsalzlösung in der angegebenen Dosierung verabreicht wurde.

**Tabelle 5**

| Präparat | Dosis des Präparates | Anzahl der Tiere | Masse des Tumors g | % der Hemmung |
|---|---|---|---|---|
| Cu(acac)₂M | 5 mg/kg | 6 | Kein Tumor | 100 |
| Melfalan | 5 mg/kg | 10 | 2,4 ± 1,1 | 49 |
| Kontrolle | | 10 | 2,8 | |

### 5. Wirksamkeit bei Leukämie

Die therapeutische Wirksamkeit wurde an Leukämietumorstämmen L-1210, P-388 und speziell auf Medikamentenresistenz erzielten Stämmen P-388 untersucht. Die Lebensdauer der Tiere wurde dabei mit 60 Tagen angesetzt. Medikamenten-resistente Tumor wurden erhalten durch aufeinanderfolgende Verpflanzung der Leukämie P-388 mit Asciteszellen, welche Mäusen entnommen wurden, die mit Rubomyzin (Stamm P388/ph), Vinkristin (P388/vcr) und Zisplastin (P388/cPt) behandelt worden waren.

Die Resistenz gegenüber den genannten Präparaten stellte sich bei der 8-ten, 6-ten und 4-ten Generation ein. Die Untersuchungen ergaben, dass die Stämme P388/ph und P388/vcr über einen Phenotyp und einen Genotyp mit multifaktorieller Medikamentenresistenz verfügten.

Die Ergebnisse zeigen die nachfolgenden Tabellen 6, 7 und 8,

### 5a. Leukämie L-1210

Inokulum: 10⁶ Zellen in 0,2 ml physiologischer Kochsalzlösung. Mäuse: BDF₁, Weibchen 19-21 g. Die Präparate wurden intraabdominös verabfolgt.

Nach Transplantation des Tumors wurde den Tieren die Substanz MOC-Melphalan intraabdominal in einer Dosierung von 5 mg/kg in 0,3 ml 10 %-iger Twin-80-Lösung an den Tagen 1 bis 7 verabreicht. Die Wirkung der Substanz wurde anhand der Lebensdauer und der Gewichtsveränderung der Tiere beurteilt (Tabelle 9). Die Beobachtungszeit betrug 60 Tage.

**Tabelle 6**

| Präparat | Einmalige Dosis mg/kg | Regime der Verabfolgung Tage | Anzahl der Tiere im Experiment | % der überlebenden Tiere | Veränderung des Gewichts g | Lebensdauer der Tiere im Versuch (Tage) |
|---|---|---|---|---|---|---|
| Cu(acac)₂ M | 5 | 1-7 | 6 | 100 | -1,5 | > 60 |
| Kontrolle | 5 | 1-7 | 6 | 0 | +0,7 | 8,5 |

### 5b. Leukämie P-388

Inokulum: 10⁶ Zellen in 0,2 ml physiologischer Kochsalzlösung. Mäuse: BDF₁. Weibchen 19-21 g

Nach Transplantation des Tumors wurde den Tieren die Substanz MOC·Melphalan intraabdominal in einer Dosierung von 5 mg/kg in 0,3 ml 10 %-iger Twin-80-Lösung an den Tagen 1 bis 7 verabreicht. Die Wirkung der Substanz wurde anhand der Lebensdauer und der Gewichtsveränderung der Tiere beurteilt (Tabelle 7).

**Tabelle 7**

| Präparat | Einmalige Dosis mg/kg | Regime der Verabfolgung Tage | Anzahl der Tiere im Experiment | Anzahl der Tiere, die bis zum 60. Tag überlebt hatten | Durch-schnittl. Lebensdauer, Tage | Zunahme der durch- schnittl. Lebensdauer % | Veränderung des Gewichtes in g |
|---|---|---|---|---|---|---|---|
| Cu(acac)₂M | 5 | 1-7 | 6 | 0 | 16,8 | 56,0 | -2,5 |
| Kontrolle | | | 6 | 0 | 10,8 | - | +1,6 |

Die Präparate wurden intraabdominal verabfolgt. Cu(acac)₂M war in 10 % Twin 80 aufgelöst.

**Tabelle 8**

| Stamm | Präparat | Dosis mg/kg | Regime (Tage nach der Verpflanzung) | ILS %* | Anzahl der überlebenden Tiere/Anzahl der Tiere in der Gruppe |
|---|---|---|---|---|---|
| P-388** o.S. (Ausgangsstamm) | Cu(acac)₂M | 5 | 1-7 | 56 | - |
| | | 10 | 1,5,9 | 419 | 5/6 |
| | | 10 | 1,7 | 465 | 4/6 |
| | | 15 | 1,7 | 447 | 5/6 |

| Medikamentenresistente Tumore | | | | | |
|---|---|---|---|---|---|
| P388/ph | Cu(acac)₂M | 5 | 1-7 | 189 | 2/6 |
| P388/vcr | | 5 | 1-7 | 516 | 5/6 |
| P388/cPt | | 5 | 1-7 | 193 | - |

| | | | | | |
|---|---|---|---|---|---|
| * mittlere prozentuale Uberlebenszeit Bei der ILS-Bestimmung wurde der Referenzwert für die Überlebensrate der Tiere auf 60 Tage festgelegt. | | | | | |
| ** o.S. - ursprünglicher Stamm, nicht resistent | | | | | |
| pH - Rubomycin-resistenter Stamm | | | | | |
| vcr - Vincristin-resistenter Stamm | | | | | |
| cPt - Cisplatin-resistenter Stamm | | | | | |

Die arzneimittelresistenten Tumore wurden durch Verabreichung von Aszites-Zellen Leukämie P-388 erhalten, die von Mäusen, welche mit Rubomycin, Vincristin und Cisplatin behandelt wurden, stammen. Die Resistenz wurde in der 4., 6. und 8. Generation gefunden. Die Empfindlichkeit der resistenten Tumore wurde durch die Anwendung der Substanz MOC"Melphala um das 4-5-fache gesenkt.

Wie aus den Tabellen 6 bis 8 zu entnehmen ist, wurden die interessantesten Resultate bei Leukämie P-388 mit multifaktorieller Medikamentenresistenz erzielt. Diese, auf zahlreiche Antitumormittel nur schwach reagierenden Tumore, waren gegenüber dem erfindungsgemäßen Präparat sensibel.

Ebenso bemerkenswert ist die Wirkung gegenüber L-1210, da alle Tiere der Versuchsgruppe 60 Tage nach der Transplantation des Tumors überlebten. Eine solche Überlebensdauer entspricht ihrer völligen Ausheilung.

### Beispiel 4: Immunmodulatorische Eigenschaften der erfindungsgemäßen Komplexe am Beispiel von (C₅H₇O₂₎₂-Cu-C₁₃H₁₈Cl₂N₂O₂

Die immunmodulatorischen Eigenschaften des erfindungsgemäßen Komplexes (C₅H₇O₂)2-Cu-C₁₃H₁₈Cl₂N₂O₂ wurden anhand der Vermehrung der Antikörper-bildenden Zellen von weißen, rasselosen Mäusen (mittleres Gewicht: 20 g) bestimmt. Die Mäuse wurden intraperitoneal mit 2 × 10⁸ Schaf-Erythrozyten in 0,2 ml physiologischer Kochsalzlösung immunisiert. Eine halbe Stunde nach erfolgter Immunisierung erhielten die Mäuse 0,3 mg/kg (C₅H₇O₂)₂-Cu-C₁₃H₁₈Cl₂N₂O₂ in 0,6 ml Olivenöl peroral verabreicht. Nach 4 Tagen wurden die Tiere getötet, die Milz wurde entnommen, in Lösungsmittel homogen suspendiert und 0,5 ml der Suspension wurden auf einer Petrischale mit Schaf-Erythrozyten auf einem Agar ausgestrichen. Die Experimente zeigten, dass (C₅H₇O₂)₂-Cu-C₁₃H₁₈Cl₂N₂O₂ in einer Dosierung von 0,3 mg/kg Körpergewicht die Anzahl der Antikörper-bildenden Zellen in der Milz der immunisierten Tiere erhöhte. So betrug die Anzahl der Antikörper bildenden Zellen der Kontrollgruppe, der nur 0,6 ml Olivenöl peroral verabreicht wurde, 76.000 ± 5.000, während die Anzahl der Antikörper-bildenden Zellen der immunisierten Tiere 158.000 ± 7.000 betrug.

### Beispiel 5: Toxizitätsuntersuchungen der erfindungsgemäßen Komplexe am Beispiel von (C₅H₇O₂)₂-CU-C₁₃H₁₈Cl₂N₂O₂

Die Toxizität des erfindungsgemäßen Komplexes (C₅H₇O₂)₂-Cu-C₁₃H₁₈Cl₂N₂O₂ wurde an fünf Labortierarten in verschiedenen Tests bestimmt. Die Resultate der Untersuchungen zeigten, dass (C₅H₇O₂)₂-Cu-C₁₃H₁₈Cl₂N₂O₂ in einer Dosierung von 5 mg/kg Körpergewicht keine schweren Veränderungen im periphären Blutbild verursacht, keine pathologischen Einflüsse auf die Nieren- und Leberfunktion hat und in den Organen und Geweben keine spezifischen Veränderungen hervorruft. Zusätzlich konnte durch die Untersuchungen gezeigt werden, dass (C₅H₇O₂)₂-Cu-C₁₃H₁₈Cl₂N₂O₂ selbst bei einer längeren Verabreichungsdauer keine Resistenzen hervorruft.

### Beispiel 6: Antitumoraktivität bei Melanom B-16

### Experiment I

Nach Transplantation des Tumors Melanom B-16 auf Mäuse der Black-Linie (Zellsuspension mit 10 × 10⁶ Zellen/ml) wurde MOC Melphalan intraabdominal in einer Dosierung von 5 mg/kg in 0,3 ml 10 %-iger DMSO-Lösung an den Tagen 3, 5 und 9 verabreicht. Die Tiere wurden am 21. Untersuchungstag getötet und histologisch, morphologisch untersucht (siehe Tabelle 9).

**Tabelle 9**

| Wirkung der Substanz MOC·Melphalan auf den Tumor Melanom B 16 Inhibierung der Tumorproliferation | | | | |
|---|---|---|---|---|
| Gruppe | Dosierung in mg/kg | Zahl der Tiere | Durch- schnittliche Tumor-masse/g | Prozentuale Hemmung |
| MOC·Melphalan | 5 | 5 | - | 100 |
| Kontrolle | - | 5 | 3,4 | - |

### Experiment II

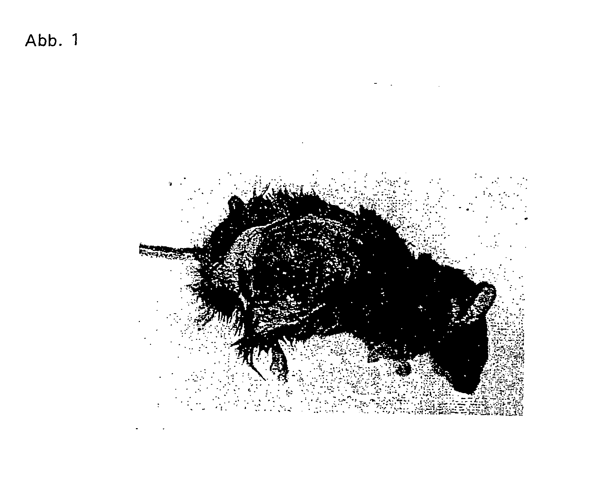

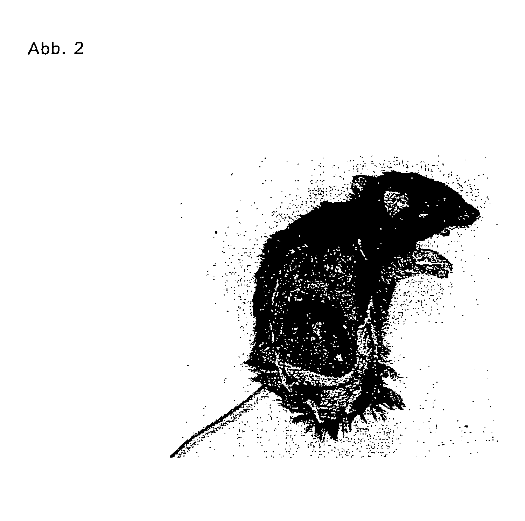

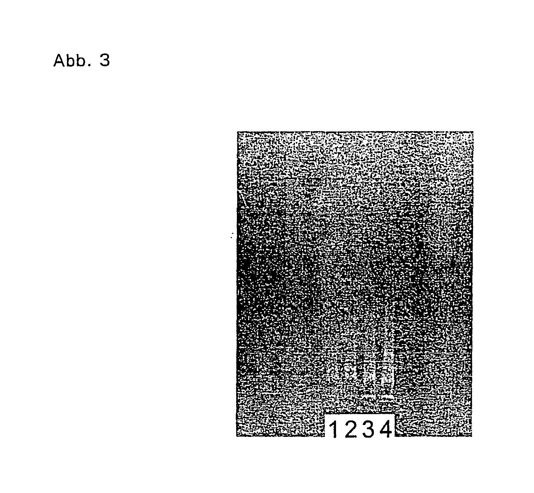

Nach Transplantation des Tumors Melanom B-16 auf Mäuse der Black-Linie (Zellsuspension mit 10 × 10⁶ Zellen/ml) wurde die Substanz intraabdominal in einer Dosierung von 0,1 mg/Tier an den Tagen 3, 5 und 9 in der 0,3 ml 10 %-iger DMSO-Lösung verabreicht. Die Tiere wurden am 16. Untersuchungstag getötet und histologisch, morphologisch untersucht (Tabelle 10, Abbildungen 1, 2). Die DNS-Konzentration in den Tumorzellen wurde spektrophotometrisch nach der Elektrophorese bestimmt. (Tabelle 10, Abbildung 3).

**Tabelle 10**

| Wirkung der Substanz MOC·Melphalan auf den Tumor Melanom B-16 Inhibierung der Tumorproliferation, Zerstörung der Tumorzell-DNS | | | | | | |
|---|---|---|---|---|---|---|
| Gruppe | Dosierung mg/Tier | Zahl der Tiere | durchschn Tumor- masse/g | prozentuale Hemmung | DNS mg/g | Mitose-index |
| MOC·Melphalan | 0,1 | 6 | 0,83 | 78 | 0,8 | 0,6 |
| Kontrolle | - | 6 | 3,22 | - | 4,3 | 4,2 |

### Beispiel 7: Aktivität bei Sarkom S-180

### Experiment I

Nach Transplantation des Tumors Sarkom S-180 auf weiße rasselose Mäuse wurde den Tieren die Substanz MOC·Melphalan intraabdominal in einer Dosierung von 1 mg/kg in 0,3 ml 10 %-iger DMSO-Lösung an den Tagen 3, 5 und 9 verabreicht. Die Tiere wurden am 21. Untersuchungstag getötet und histologisch, morphologisch untersucht (Tabelle 11).

**Tabelle 11**

| Wirkung der Substanz MOC·Melphalan auf den Tumor Sarkom S-180 Inhibierung der Tumorproliferation | | | | |
|---|---|---|---|---|
| Gruppe | Dosierung in mg/kg | Zahl der Tiere | durchschn. Tumormasse/ g | prozentuale Hemmung |
| MOC·Melphalan | 1 | 6 | - | 100 |
| Kontrolle | - | 6 | 4,7 | - |

### Experiment II

Nach Transplantation des Tumors Sarkom S-180 auf weiße rasselose Mäuse wurde den Tieren MOC·Melphalan und Melphalan intraabdominal in einer Dosierung von 5 mg/kg in 0,3 ml 10 %-iger DMSO-Lösung an den Tagen 3, 5, 7 und 9 verabreicht. Die Tiere wurden am 21. Untersuchungstag getötet und histologisch, morphologisch untersucht (Tabelle 12).

**Tabelle 12**

| Wirkung der Substanz MOC·Melphalan auf den Tumor Sarkom S-180 Inhibierung der Tumorproliferation | | | | |
|---|---|---|---|---|
| Gruppe | Dosierung in mg/kg | Zahl der Tiere | durchschn. Tumormasse/g | prozentuale Hemmung |
| MOC·Melphalan | 5 | 6 | - | 100 |
| Melphalan | 5 | 10 | 1,4 | 49 |
| Kontrolle | - | 10 | 2,8 | - |

### Experiment III

Nach Transplantation des Tumors Sarkom S-180 auf weiße rasselose Mäuse wurde den Tieren MOC·Melphalan und Melphalan intraabdominal in einer Dosierung von 5 mg/kg in 0,3 ml 10 %-iger DMSO-Lösung an den Tagen 2, 4, 6, 8 und 10 verabreicht. Die Tiere wurden am 21. Untersuchungstag getötet und histologisch, morphologisch untersucht (Tabelle 13).

**Tabelle 13**

| Wirkung der Substanz MOC·Melphalan auf den Tumor Sarkom S-180 Inhibierung der Tumorproliferation | | | | |
|---|---|---|---|---|
| Gruppe | Dosierung in mg/kg | Zahl der Tiere | durchschn. Tumormasse/g | prozentuale Hemmung |
| MOC·Melphalan | 5 | 10 | 2,0 ± 0,7 | 49 |
| Melphalan | 5 | 10 | 2,4 ± 1,1 | 38 |
| Kontrolle | - | 20 | 3,9 ± 0,5 | - |

### Beispiel 8: Wirkung am Adenokarzinom des Dünndarms (AKATON)

### Experiment I

Nach Transplantation des Tumors AKATON auf weiße rasselose Mäuse wurde den Tieren die Substanz MOC·Melphalan intraabdominal in einer Dosierung von 5 mg/kg in 0,3 ml physiologischer Lösung an den Tagen 3, 5, 7 und 9 verabreicht. Die Tiere wurden am 21. Untersuchungstag getötet und histologisch, morphologisch untersucht (Tabelle 14).

**Tabelle 14**

| Wirkung der Substanz MOC·Melphalan auf den Tumor AKATON Inhibierung der Tumorproliferation bei intraabdominaler Applikation | | | | |
|---|---|---|---|---|
| Gruppe | Dosierung in mg/kg | Zahl der Tiere | durchschn. Tumormasse | prozentuale Hemmung |
| MOC·Melphalan | 5 | 10 | 0,3 | 92 |
| Kontrolle | - | 10 | 3,6 | |

### Experiment II

Nach Transplantation des Tumors AKATON auf weiße rasselose Mäuse wurde den Tieren die Substanz MOC-Melphalan intravenös in einer Dosis von 5 mg/kg in 0,3 ml physiologischer Lösung an den Tagen 3, 5, 7 und 9 verabreicht. Die Tiere wurden am 21. Untersuchungstag getötet und histologisch, morphologisch untersucht (Tabelle 15).

**Tabelle 15**

| Wirkung der Substanz MOC·Melphalan auf den Tumor AKATON Inhibierung der Tumorproliferation bei intravenöser Applikation | | | | |
|---|---|---|---|---|
| Gruppe | Dosierung in mg/kg | Zahl der Tiere | durchschn. Tumormasse/ g | prozentuale Hemmung |
| MOC·Melphalan | 5 | 6 | 0,9 | 80 |
| Kontrolle | - | 6 | 4,4 | - |

### Experiment III

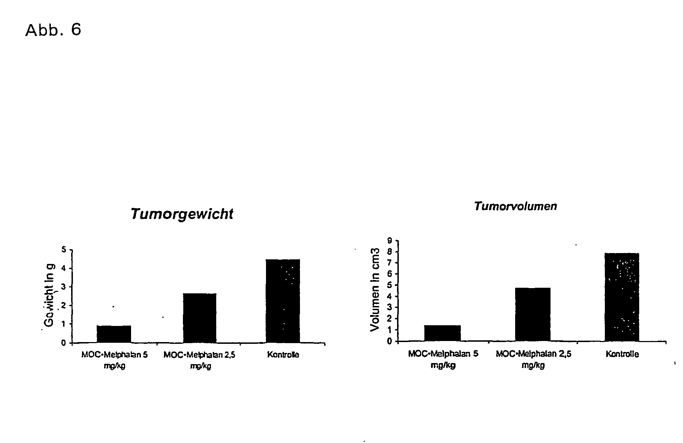

Untersuchung zur Tumorprävention der Substanz MOC·Melphalan Mäusen der Balb-Linie wurde die Substanz MOC·Melphalan intravenös in Dosierungen von 2,5 mg/kg und 5 mg/kg in 0,3 ml physiologischer Lösung an vier aufeinanderfolgenden Tagen (1 × töglich) verabreicht. Am Tag 5 wurde den Tieren der Tumor AKATON implantiert. Die Tiere wurden am 21. Untersuchungstag getötet und histologisch, morphologisch untersucht (Tabelle 16, Abbildung 6).

**Tabelle 16**

| Präventive Wirkung der Substanz MOC·Melphalan auf den Tumor AKATON - Inhibierung der Tumorproliferation | | | | | | |
|---|---|---|---|---|---|---|
| Gruppe | Dosierung mg/kg | Zahl der Tiere | durchschn . Tumor- masse/g | prozentuale Hemmung des Tumorgewichts | durchschn. Tumor-volumen/cm³ | prozentuale Hemmung des Tumor-volumens |
| MOC·Melphalan | 5,0 | 6 | 0,91 | 80 | 1,38 | 82 |
| MOC·Melphalan | 2,5 | 6 | 2,67 | 40 | 4,76 | 40 |
| Kontrolle | - | 6 | 4,46 | - | 7,90 | - |

### Beispiel 9: Bestimmung der DNA-Konzentration in Zellen von Mäusen mit Sarkom S-180 nach Einwirkung der Substanz MOC·Melphalan

Tumorzellen des Sarkom S-180 (20·10⁶ Zellen/Untersuchung) wurden 40 bzw. 60 min mit der Substanz MOC·Melphalan in einer Konzentration von 0,05 mg/10⁶ Zellen, bei einer Temperatur von 37 °C inkubiert (siehe Tabelle 16).

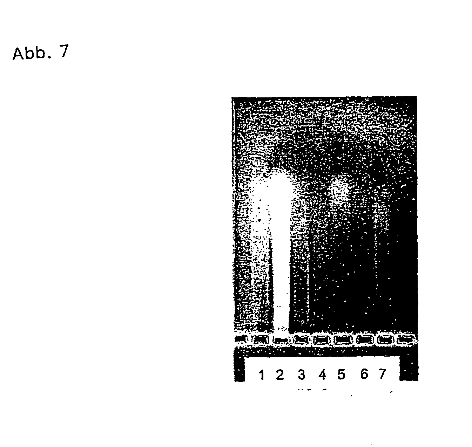

Die DNA-Konzentration wurde mit der Phenol-Chloroform-Methode nach MANIATIS bestimmt (Maniatis, Frin, Sämbruck Methoden der genetischen Technologie, Molekulare Klonierung, M.: MIR, 1984, Seite 479 ff.). Nach der Trennung von DNA und RNA wurde eine Phorese in 2,5 %-igem Agarosegel durchgeführt (siehe Tabelle 16, Abbildung 7). Die Menge der DNA wurde aus dem Verbrauch von RN-ase berechnet, d.h. die Daten geben die genaue Masse der DNA an.

**Tabelle 17**

| Einfluss der Substanz MOC·Melphalan auf die DNA-Konzentration in *Tumorzellen des Sarkom* S-180 | | | | |
|---|---|---|---|---|
| Gruppe | Inkubationszeit /*min* | Dosierung mg/10⁶ Zellen | DNA- Konzentration µg/g Zellen | DNA-Konzentration/ Kontrolle' |
| MOC-Melphalan | 40 | 0,05 | 530 | 498 |
| MOC·Melphalan | 60 | 0,05 | 53 | 50 |
| Kontrolle | - | - | 106 | 100 |

### Beispiel 10: Synthese des Komplexes Cu (acac)₂-Tegafur

Der Komplex Cu(acac)₂·Tegafur wurde durch langsame Auskristallisation aus einer Chloroform-Methanol-Lösung, angesäuert mit Salzsäure, erhalten.

*Herstellung:* 2,61 g (1 × 10⁻² mol) Kupfer-Acetylacetonat Cu(C₅H₇O₂) werden in 50 ml gereinigtem Chloroform gelöst. Die Lösung hat eine dunkelblaue Farbe. 4 g (2 × 10⁻² mol) Tegafur (N¹-(2-Furanidil)-5-Flururacil) werden in 50 ml Chloroform-Methanol-Lösung im Verhältnis 1:1 1 gelöst. Die erhaltenen Lösungen wurden im Wasserbad bis zum Sieden erhitzt und unter ständigem Rühren mit Hilfe eines Magnetrührers zusammengegeben. Die Lösung nimmt eine leuchtend grüne Farbe an. Das Glasgefäß mit der Lösung wird an einen dunklen Platz zur langsamen Auskristallisation deponiert. 3 bis 4 Tage nach der vollständigen Verdampfung des Lösungsmittels wird der braun-grüne Rückstand mit Chloroform bis zum vollständigen Auswaschen des nicht reagierten Cu(C₅H₇O₂)₂ und Tegafur gereinigt. Die zurückgebliebenen grünen Kristalle werden an der Luft getrocknet.

*Aufbau und Struktur:* des Komplexes MOC·Tegafur wurden anhand der Methoden EPR, NMR, Elektronen- und Infrarotspektroskopie durchgeführt.

### Charakterisierung der Verbindung MOC·Tegafur

Das Produkt stellt einen polykristallinen, metallorganischen Komplex dar, der eine grüne Farbe besitzt. Das stöchiometrische Verhältnis Cu(C₅H₇O₂)₂:Tegafur beträgt 1:1. Die Molekularmasse des Komplexes Cu(acac)₂·Tegafur beträgt 461,2 g/mol, der Komplex löst sich gut in Wasser, in physiologischer Lösung, in Methanol, Ethanol, DMSO und Twin-80. Die Verbindung ist nicht löslich in Ether und Chloroform. Die Schmelztemperatur der Kristalle beträgt 127 °C. Die Verbindung ist an der Luft über 5 Jahre stabil.

### Spektroskopische Parameter von MOC·Tegafur

UV-Spektren (Lösung: Methanol-Chloroform, Verhältnis: 1:3):
- UV-VIS-Spektrum:: - UV-Bereich: Intensitätslinien bei 36200 cm⁻¹ (276 nm)
- VIS-Bereich: charakteristischer Übergang von dₓ²_{-y}² → d_{xy} bei λ = 12345 cm⁻¹ (810 nm)
ESR-Spektren: Methanol-Chloroform-Lösung im Verhältnis 1:3 bei Raumtemperatur und Temperatur von 77 °K:
g_{II} = 2,301, g_{┴} = 2,05, g₀ = 2,146
A_{II} = 160·10⁻⁴ cm⁻¹ A_{┴} = 14·10⁻⁴ cm⁻¹ A₀ = 52·10⁻⁴ cm⁻¹
NMR-Spektren in deuteriertem Methanol-Chloroform (1:3): In der Tabelle sind die Werte der chemischen Verschiebung der Protonen im NMR-Spektrum des Komplexes MOC·Tegafur und des reinen Tegafur zum Vergleich (a, b, c, d, f entsprechen der Protonenlage) angegeben. 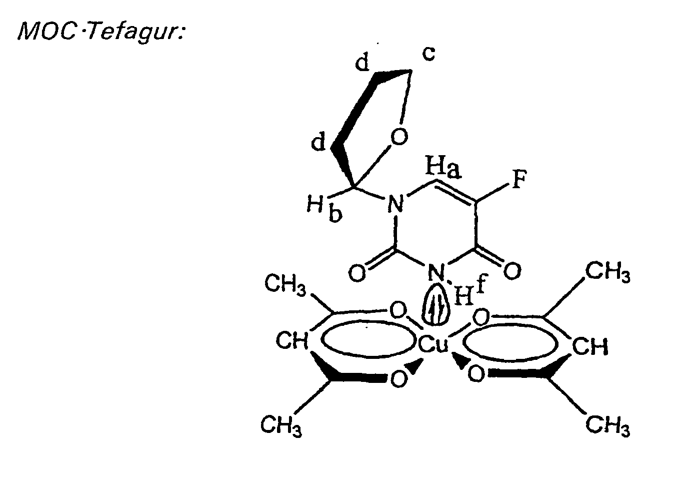 Werte der chemischen Verschiebung der Protonen bei den Substanzen Tegafur und MOC·Tegafur

**Tabelle 18**

| Substanz | a | b | c | g | f |
|---|---|---|---|---|---|
| Tegafur | 7,8 | 5,8 | 3,7; 4,1 | 2,0 | - |
| MOC·Tegafur | 7,82 | 5,86 | 4,15 | 1,9 | 11,73 |

### Beispiel 11: Antitumorwirkung von MOC·Tegafur

### Experiment I:

48 Stunden nach der Transplantation des Tumors AKATON (Adenokarzinom Dünndarm) wurde den Mäusen der Balb-Linie die Substanz MOC·Tegafur intraabdominal in 0,3 ml einer physiologischen Lösung in einer Dosierung von 5 mg/kg Körpergewicht verabreicht. Dies erfolgte am 3., 5., 7. und 9. Tag. Die Tiere wurden am 21. Untersuchungstag getötet.

**Tabelle 19**

| Einfluss der Substanzen MOC·Tegafur und Tegafur auf den Tumor AKATON (viermalige Verabreichung der Substanzen) | | | | |
|---|---|---|---|---|
| | Dosis (mg/kg) | Anzahl der Tiere | Tumorgewicht (g) | prozentuale Hemmung* |
| MOC·Tegafur | 5 | 10 | 0,61 | 86 |
| Tegafur | 250 | 10 | 1,95 | 54,5 |
| Tumorkontrolle | - | 10 | 4,4 | - |

| | | | | |
|---|---|---|---|---|
| * im Vergleich zur Kontrollgruppe | | | | |

### Experiment II:

48 Stunden nach der Transplantation des Tumors AKATON wurde den Mäusen der Balb-Linie die Substanz MOC·Tegafur intravenös in 0,3 ml einer physiologischen Lösung verabreicht. Dies erfolgte am 3., 5., 7. und 9. Tag. Die Tiere wurden am 21. Untersuchungstag getötet.

**Tabelle 20**

| Einfluss der Substanzen MOC·Tegafur und Tegafur auf den Tumor AKATON bei intravenöser Verabreichung (viermalige Verabreichung der Substanzen) | | | | |
|---|---|---|---|---|
| | Dosis (mg/kg) | Anzahl der Tiere | Tumorgewicht (g) | prozentuale Hemmung* |
| MOC·Tegafur | 5 | 10 | 0,86 | 80 |
| Tegafur | 250 | 10 | 2,1 | 51 |
| Tumorkontrolle | - | 10 | 4,3 | - |

| | | | | |
|---|---|---|---|---|
| * im Vergleich zur Kontrollgruppe | | | | |

### Experiment III:

48 Stunden nach der Transplantation des Tumors Sarkom-180 wurde Mäusen der Balb-Linie die Substanz MOC·Tegafur intraabdominal in 0,3 ml einer physiologischen Lösung verabreicht. Die Verabreichung erfolgte am 3., 5. und 9. Tag. Die Tiere wurden am 21. Untersuchungstag getötet.

**Tabelle 21**

| Einfluss der Substanzen MOC·Tegafur und Tegafur auf den Tumor Sarkom-180 (dreimalige Verabreichung der Substanzen) | | | | |
|---|---|---|---|---|
| | Dosis (mg/kg) | Anzahl der Tiere | Tumorgewicht (g) | prozentuale Hemmung^{*} |
| MOC·Tegafur | 3 | 6 | 0,43 | 89 |
| MOC·Tegafur | 5 | 6 | 0,195 | 95 |
| Tegafur | 250 | 10 | 3,1 | 20,5 |
| Kontrolle | - | 6 | 3,9 | - |

| | | | | |
|---|---|---|---|---|
| * im Vergleich zur Kontrollgruppe | | | | |

### Beispiel 11: Untersuchung des Einflusses von MOC·Tegafur auf die AKATON-Tumorproliferation nach der Transplantation des Tumors

Das Präparat wurde intravenös in einer physiologischen Lösung von 0,3 ml 4 Tage hintereinander verabreicht (Tabelle 20). Danach wurde der Tumor AKATON transplantiert.

**Tabelle 22**

| Einfluss von MOC·Tegafur auf die Tumorproliferation bei den Untersuchungen am AKATON | | | | | | |
|---|---|---|---|---|---|---|
| | Dosis (mg/kg) | Anzahl der Tiere | Tumor- gewicht (g) | Prozentuale Hemmung des Tumor- gewichts | Größe des Tumors (cm³) | Prozentuale Hemmung des Tumor-volumens |
| MOC·Tegafur | 5 | 6 | 2,20 | 50* | 3,06 | 61,3* |
| Kontrolle | - | 6 | 4,46 | - | 7,90 | - |

| | | | | | | |
|---|---|---|---|---|---|---|
| * im Vergleich zur Kontrollgruppe | | | | | | |

### Beispiel 12: Strukturmorphologische Änderungen im Tumorgewebe bei Mäusen mit Sarkom-180 durch Einwirkung von MOC·Tegafur

Geschlechtsreifen rasselosen Mäusen wurde der Tumor Sarkkom-180 mit einem Gewicht von 20 bis 22 g transplantiert. 48 Stunden nach der Transplantation des Tumros Sarkom-180 wurde den Mäusen die Substanz MOC·Tegafur intraabdominal in 0,9 % NaCl-Lösung in einer Dosierung von 5 mg/kg Körpergewicht verabreicht. Die Verabreichung erfolgte am 3., 5. und 9. Tag.

Am 21. Tag wurden die Mäuse durch Dekapitation getötet und das Tumorgewebe für die histologische Untersuchung entnommen. Die Antitumoraktivität von MOC·Tegafur wurde vorläufig bestimmt. Die prozentuale Hemmung der Tumorproliferation bei dieser Untersuchung betrug 96,4 %.

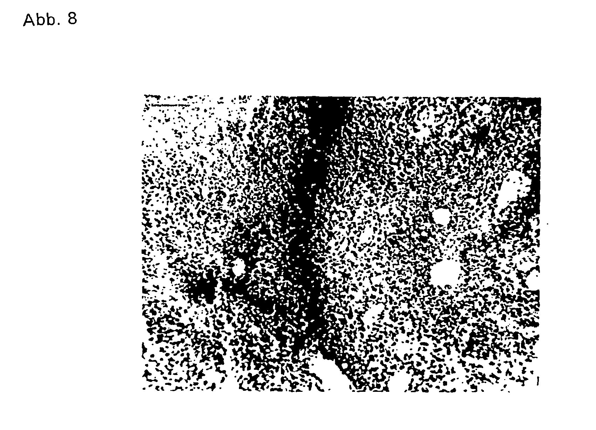

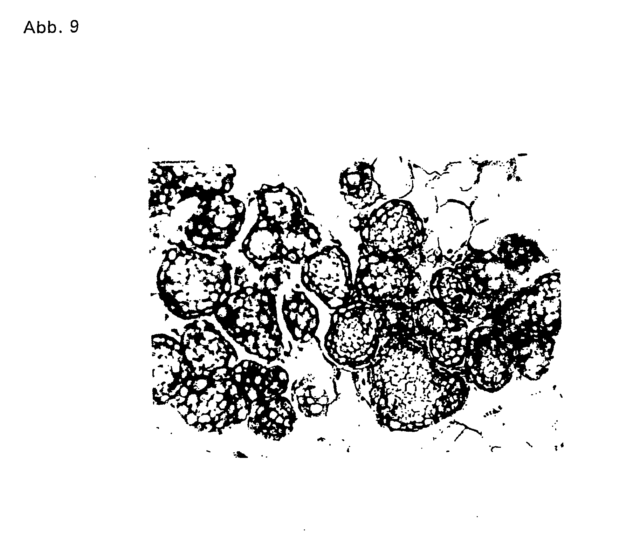

Die morphologische Struktur der histologischen Tumorschnitte bei den Tieren, die MOC·Tegafur erhielten, unterscheidet sich von den Tumoren der Tiere in der Kontrollgruppe. Zum einen bildet sich zwischen dem gesunden und Tumorgeweben eine doppelschichtige Hülle, die mit einer Kapsel vergleichbar ist. Außen besteht sie aus Muskelfasern, im Inneren sind Zellen mit Membranen zu erkennen. Die Zellen sind meist ohne Inhalt (Abbildung 8). Zweitens ist eine große Zahl von Hohlräumen verschiedener Größen vergleichbar mit Vakuolen zu erkennen (Abbildung 9).

Im Tumorgewebe der mit MOC behandelten Tiere sind hochdifferenzierte Zellen und nekrotische und postnekrotische Zonen zu beobachten, sowie Blutgefäße, die eine große Zahl an Zellen enthalten. Die Tumorzellen sind meist rund mit kleinen Kernen (1 oder 2 Kerne) und enthalten kleinkerniges, kleinvernetzte, diffus verteiltes Chromatin. Wenige der großen Blastzellen sehen rund aus. Das Zytoplasma, das den großen Kern umgibt, ist nicht gleichmäßig verteilt. Das zahlreiche, umschlingende Chromatin konzentriert sich auf die Kernperipherie. Die Anzahl der mehrkernigen Zellen weist auf eine Störung der Zytokinese hin. In den blasttransformierten Zellen ist eine frühzeitige Kondensation des Chromatins zu beobachten.

Bei der Untersuchung wurde die Aktivität des Tumorgewebes bei Tieren, die das Präparat MOC·Tegafur erhielten und bei unbehandelten Tieren in der Kontrolle bestimmt.Es wurde die Anzahl von Mitosen und Mitoseindex berechnet. Die mittlere Zahl der Mitosen in der Untersuchungsgruppe betrug 2,55 % und in der Kontrolltiergruppe 11,2 %. Der Mitoseindex in diesen Tiergruppen betrug 0,9 und 4,75. Bei der Untersuchung wurde unter dem Mikroskop eine Zunahme der Zahl an pathologischen Metaphasen und Anaphasen beobachtet.

Zusammfassend ergibt sich daraus, dass das Präparat MOC·Tegafur eine große destruktive Veränderung im Tumorgewebe hervorruft.

### Beispiel 13: Einfluss von MOC-Melphalan und MOC-Tegafur auf den Tumor Melanom B-16

48 Stunden nach der Transplantation des Tumors Melanoms B-16 bei Mäuse der Linie Black wurden die Substanzen MOC·Melphalan und MOC·Tegafur in der Dosierung von 3 mg/kg Körpergewicht intraabdominal (im Folgenden an den Tagen: 3, 5 und 9) verabreicht. Unter Berücksichtigung der schlechten Wasserlöslichkeit von MOC·Melphalan wurden es in 10 % DMSO-Lösung (aufgefüllt mit 0,15 molarer NaCl) gelöst. MOC·Tegafur wurde in einer physiologischen Lösung verabreicht. Den Tieren der Kontrollgruppe wurden die Lösungen ebenfalls verabreicht (ohne Präparate). Am 16. Tag wurden die Mäuse durch Dekapitation getötet, Tumorgewebe für die histologische Untersuchung entnommen und der Tumor auf Antitumoraktivität untersucht (Tabelle 23). Die Fixierung der Tumore erfolgte mit 10 %-Formalin und danach wurde eine Paraffineinbettung durchgeführt. Die Schnittdicke von 5 µm wurden mit Hämatoxylin-Eosinom gefärbt. Die Mikroskopie wurde mit einem Leica Galen-Mikroskop durchgeführt.

Die Untersuchungsergebnisse dieser Verbindungen, die in der Tabelle 23 zusammengestellt sind, deuten auf eine hohe Antitumoraktivität hin.

**Tabelle 23**

| | Anzahl der Tiere | Dosis mg/kg | Tumor- gewicht g | prozentuale Hemmung * | Ml (Mitose -index) |
|---|---|---|---|---|---|
| MOC·Tegafur | 5 | 5,0 | 1,13 | 65,66 | 0,86 |
| MOC·Melphalan | 5 | 5 | 0,74 | 77,71 | 0,64 |
| Kontrolle | 5 | | 3,22 | | 4,22 |

| | | | | | |
|---|---|---|---|---|---|
| * im Vergleich zur Kontrollgruppe | | | | | |

Bei der Einwirkung auf den Tumor wirken die Substanzen im logarithmischen Proliferationsstadium nicht nur auf die Größe und das Gewicht der Tumore, sondern sie inhibieren außerdem die Teilungsprozesse und die Lebensfähigkeit der Tumorzellen.

Bei den Tiergruppen, die die Substanzen MOC·Melphalan und MOC·Tegafur erhielten, wurde eine Hemmung der Tumorproliferation beobachtet, aber auch die Anzahl der Mitosezellen war geringer (Tabelle 21).

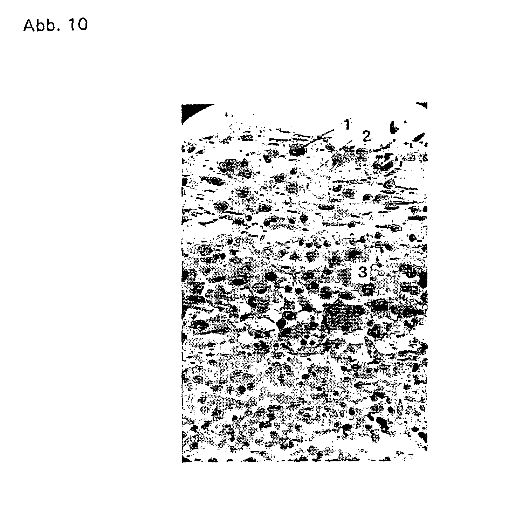

Morphologisch sind diese Wirkungsbesonderheiten der Verbindungen deutlich zu sehen. Bei der Mikroskopie der Tiertumore (die Tiere, die MOC·Tegafur erhalten haben (Abbildung 10)) ist eine Kapselformmierung zu beobachten. Innerhalb der Kapseln sind verschiedene Zelltypen erkennbar: zum einen epithelähnliche Zellen mit der Degradation und zum anderen Muskelzellen. Weiterhin ist Tumorgewebe zu erkennen. Im Tumorgewebe ist eine große Anzahl an nekrosen Zonen zu beobachten. Die Tumorzellen sind hochdifferenziert, mit verschiedenen Zellkernen. Das Chromatin ist meist großkernig, kleinvernetzt, man kann auch Zellen mit viel Chromatin sehen. Die Zahl der teilbaren Zellen ist gering (Ml beträgt 0,86).

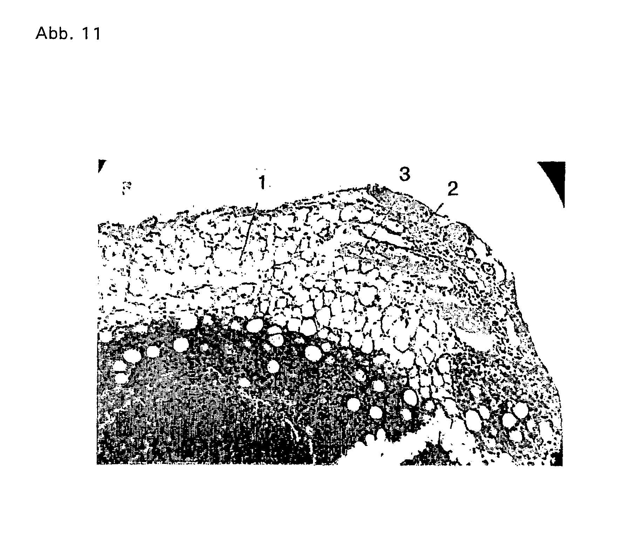

Ein anderes Bild der morphologischen Struktur ist im Tumorgewebe bei den Tieren zu beobachten, die MOC·Melphalan erhalten haben (Abbildung 11). Die Oberschicht der Kapsel ist in diesem Fall wesentlich größer, gleichartiger und besteht aus den Parenchymfettzellen. Gleichartige Zellen sind an der Vorgrenze vom Tumorgewebe zu erkennen. Im Zentrum des Tumors sind kerotische Abschnitte und Streifen zu beobachten. Man kann auch die Zerstörungen bei den Interzellkontakten sehen. Die Tumorzellen haben meist wenig Zytoplasma, die Kerne sind verzerrt und enthalten ein körniges Chromatin. Die Zahl der teilbaren Zellen ist gering. Man erkennt eine kleine Zahl mit großen transformierten Kernen. Im Tumorgewebe sind auch alveoläre Strukturen vorhanden, die sich aus den antitypischen_ melanoblastomen-epithelähnlichen Typen formieren.

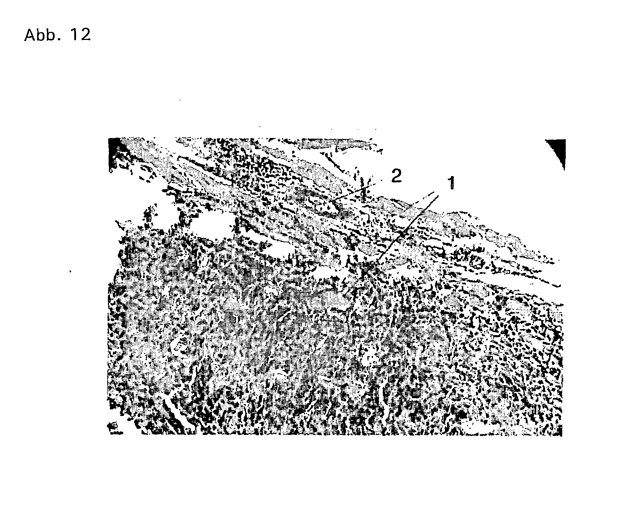

Bei den Tiertumoren der Kontrollgruppe bleibt die Kapselbildung aus (Abbildung 12). Die Außenschicht besteht aus Muskelbündeln, die sich in der Mehrzahl im Tumorgewebe befinden. Die meisten aktiv teilbaren Tumorzellen zeigen überwiegend einen normalen Mitoseverlauf. Die Kerne sind groß und enthalten verschiedenartiges Chromatin. Kleine nekrotische Zellen trifft man selten.

Die vergleichende Analyse der morphologischen Bilder der drei Tiergruppen lässt darauf schließen, dass die erfindunsgemäßen Komplexe über eine gezielte Wirkung auf die Tumorgewebe verfügen. Trotz der Unterschiede in der morphologischen Struktur, der Einwirkungstendenz der Präparate auf die Tumordegradation ist folgendes Gleich: Verkapselung, wesentliche Degradation des Chromatins, Gewebeaufteilung, Senkung der proliferativen Zellaktivität.

### Beispiel 14: Einwirkung auf Zellkulturen

Es wurde die Wirkung der erfindungsgemäßen Substanzen MOC·Melphalan und MOC·Tegafur auf die proliferative Aktivität, Morphologie und Proteinsynthese an den Tumorzellkulturen (KML) untersucht. Dies erfolgte bei den aus der Linie Melanom-16 ausgesonderten Mäusen. 80.000 Zellen/ml wurden in 3 ml DMEM mit dem Zusatz von embryonalem Kälberserum, 200 µ/mol des Glutamine und Antibiotikum, verteilt. Nach der Zellkultivierung von 24 Stunden bei einer Temperatur von 37 °C (Logarithmische Zellen-Wachstums-Phase) wurden die MOC-Substanzen in der Konzentration von 10 und 100 *µ*g/ml zugegeben. Nach der 24-stündigen Inkubation wurde die Zahl der lebendigen Zellen bestimmt. Parallel wurden die fixierten Präparate der Zellen für die morphologische Analyse vorbereitet.

Alle zwei MOC-Substanzen in der Konzentration von 100 µg/ml rufen die Unterdrückung des Tumorwachstums (Tabelle 24) und ein weiteres Absterben der Tumorzellen hervor.

**Tabelle 24**

| Untersuchungen zur Zellproliferationsunterdrückung durch Einwirkung von MOC·Tegafur und MOC·Melphalan | | |
|---|---|---|
| | Dosis (µg/ml) | Unterdrückung* der Wachstumszellen (%) |
| MOC·Tegafur | 100 | 100 |
| | 10 | 32 |
| MOC·Melphalan | 100 | 95 |
| | 10 | 30 |

| | | |
|---|---|---|
| * im Vergleich zur Tumorkontrolle | | |

Die morphologische Analyse hat gezeigt, dass bei der Dosierung von 10 µg/ml MOC·Tegafur eine Ansammlung von Hyperchromie- und pyknotischen Zellen hervorruft. MOC·Melphalan führt zu der Zellenbildung mit Zytoplasma, ähnlich einer Vakuole.

Die Wirkung der Substanzen auf die Proteinsynthese bestimmt man mit Hilfe des Unterdrückungsstadiums durch den Einschluss der radioaktiven Präkursoren der Aminosäuren (summierte Mischung ³H-Aminsäuren). Dafür wurden 1 Million Zellen, die sich in einer "log-Phase" befinden, ausgewählt. In die 10 ml Flaschen wurden die Komplexe in der Dosierung von 50 *µ*g/ml und parallel die Aminosäuren mit einer Aktivität von 10 mCᵢ/ml eingetragen. Nach der 24-stündigen Inkubation wurden die Zellen von dem Kultivierungsnährboden ausgewaschen und eine Lysis (Auflösung) durchgeführt. Die Radioaktivität der Proteine wurde mit dem Zellzählgerät bestimmt (Tabelle 25).

**Tabelle 25**

| Wirkung von MOC·Tegafur und MOC·Melphalan auf die Proteinbiosynthese | | |
|---|---|---|
| | Dosis (µg/ml) | Unterdrückung des Aminosäureeinschlusses (%) |
| MOC·Tegafur | 50 | 77 |
| MOC·Melphalan | 50 | 59 |

| | | |
|---|---|---|
| * im Vergleich zur Kontrolle (Tumorzellkulturen) | | |

Die Senkung der Proteinsynthese in den Zellen bei den untersuchten Gruppen wurde im Vergleich zu der Kontrollgruppe der Zellen bestimmt. Wie aus Tabelle 25 ersichtlich ist, inhibiert besonders MOC·Tegafur die Proteinbiosynthese.

Die Einwirkung der MOC-Substanzen auf die Tumorzellkulturen mit Melanom B-16 führt zur Änderung der morphologischen Struktur, Senkung der proliferativen Aktivität und Inhibierung der Proteinsynthese. Diese wird durch die Untersuchungsergebnisse bestätigt, die zuvor durch Einwirkung der Komplexe auf den Tumor B-16 bei den Mäusen erhalten wurden.

### Beispiel 15: Pharmakokinetik der Speicherung von MOC-Präparaten in den Geweben des Organismus

Die Chemotumortherapie verlangt eine selektive Wirkung von Antikrebspräparaten. Zu der Selektivität kann sie Speicherungsfähigkeit der Präparate im Tumorgewebe gerechnet werden. Für die Bestimmung wurden speziell markierte ³H-Cu(acac)₂ mit Tegafur synthetisiert. Das markierte Präparat wurde sorgfältig mit Hilfe eines Chromatographen von Begleitstoffen gesäubert. Die gesamte Radioaktivität des ³H-Cu(acac)₂Ft beträgt 0,16 Mikroeinheiten.

Die Verteilung und Speicherung des gekennzeichneten Komplexes in den Organen und Geweben wurde an Mäusen mit transplantiertem AKATON (Dünndarmkrebs) untersucht. Das Präparat wurde in einer Dosierung von 1.200.000 Impulsen pro Minute am 13. Tag nach der Tumortransplantation verabreicht. Es wurden drei Gruppen von Mäusen von je 5 Stück gebildet. Die Tiere wurden 30, 60 und 180 Minuten nach der Präparat-Verabreichung getötet und anhand der radioaktiven Impulse mit einem β-Zähler wurden die Organe untersucht (β-Szintigraphie). Die Ergebnisse sind in Tabelle 26 dargestellt.

**Tabelle 26 Speicherungsverlauf des ³H-Cu(acac)₂·Tegafur in Organen und Geweben bei den untersuchten Mäusen (Impulsanzahl pro Minute)**

| Organe | 30 min | 60 min | 180 min |
|---|---|---|---|
| Gehirn | 600 | 1200 | 500 |
| Herz | Spuren | Spuren | - |
| Lunge | 2300 | 2800 | 3000 |
| Leber | 1700 | 3550 | 4000 |
| Niere | Spuren | Spuren | 1200 |
| Dünndarm | Spuren | Spuren | 3200 |
| Milz | 1400 | 1200 | 1900 |
| Blut | 3300 | 4700 | 1200 |
| Tumor | 1200 | 1800 | 12.000 |

Die maximale Speicherung des Präparates wird im Tumor beobachtet, dies belegt einen gezielten Transport im Organismus.

## Patentansprüche

1. Verwendung einer pharmazeutischen Zusammensetzung enthaltend mindestens einen Komplex der allgemeinen Formel
D₂-M-T,
worin
D ein β-Diketon darstell,
M ein Metallatom, ausgewählt aus der Gruppe, bestehend aus Cr, Cu, Mn, Fe, Ni, Co, Zn und Mo darstellt,
T eine Substanz mit mindestens einer N-, O- oder S-enthaftenden Gruppe darstellt und
wobei M mit T in einer Elektronen-Donor-Akzeptor-Wechselwirkung steht und M im Komplex eine freie Koordinationsstelle aufweist, zur Herstellung eines Antitumormittels.

2. Verwendung einer pharmazeutischen Zusammensetzung nach Anspruch 1,
**dadurch gekennzeichnet,**
**dass** D ausgewählt ist aus Acetylaceton und Dibenzoylmethan.

3. Verwendung einer pharmazeutischen Zusammensetzung nach Anspruch 1 oder 2,
**dadurch gekennzeichnet,**
**dass** T eine Substanz mit mindestens einer NH₂-, NH-, N-, O- oder S-Gruppe ist.

4. Verwendung einer pharmazeutischen Zusammensetzung nach einem der Ansprüche 1 bis 3,
**dadurch gekennzeichnet**
**dass** T eine Substanz, ausgewählt aus der Gruppe, bestehend aus 2,4-Dihydroxy-5-fluorpyrimidin, 5-Fluor-1-(tetrahydro-2-furyl)-uracil, 2-[Bis-(2-chlorethyl)-aminol-tetrahydro-2H-1,3,2-oxazephosphorin-2-oxid, 1,2-Imidopropylsäureamid, 2-Hydroxymethyl-5-hydroxy-γ-pyrone, 2,4,6-Trimethylpyridin, 2,4,6-Tri-2-pyridyl-1,3,5-triazin, 4-[Bis-(2-chlorethyl)-amino]-L-phenylalanin, 2-(3-Pyridyl)-piperidin, 2-2'-Bipyridin, 2-Methyl-(5-trimethyl-butyl-1-il-ol-3)-pyridin, 2-Methyl-(3-dimethyl-amino-1-propinyl)-pyridin und 2-Methyl-5-ethylen-pyridin, ist.

5. Pharmazeutische Zusammensetzung der allgemeinen Formel
D₂-M-T,
worin D Acetylaceton, M Cu und T eine Substanz, ausgewählt aus der Gruppe, bestehend aus 2,4-Dihydroxy-5-fluorpyrimidin, 5-Fluor-1-(tetrahydro-2-furyl)-uracil, 2-[Bis-(2-chlorethy)-amino]-rtetrahydro-2H-1,3,2-oxazephosphorin-2-oxid, 1,2-Imidopropytsäureamid, 2-Hydroxymethyl-5-hydroxy-y-pyrone,2,4,6-Trimethylpyridin, 2,4,6-Tri-2-pyridyl-1,3,5-triazin, 4-[Bis-(2-chlorethy)amino]-L-phenylalanin, 2-(3-Pyridyl)-piperidin, 2-2'-Bipyridin, 2-Methyl-(5-trimethyl-butyl-1-il-ol-3)-pyridin,2-Methyl-(3-dimethyl-amino-1-propinyl)-pyridin und 2-Methyl-5-ethylen-pyridin, ist.

6. Pharmazeutische Zusammensetzung der allgemeinen Formel
D₂ - M - T
worin D Aoetylaoeton, M Mn und T eine Substanz, ausgewählt aus der Gruppe, bestehend aus 2,4,6-Trimefhylpyridin, 2,4,6-Tri-2-pyridiyl-1,3,5-triazin, 2-2'-Bipyridin, 2-(3-Pyridyl)-piperidin. 1,2-Imidopropylsäureamid und 4-[Bis(2-cholorethyl)-amino]-L-henylalanin, ist

7. Komplex der allgemeinen Formel
D₂ - M -T,
worin D Acetylaceton, M Cu und T eine Substanz, ausgewählt aus der Gruppe, bestehend aus 2.4-Dihydroxy-5-fluorpyrimidin. 5-Fluor-1-(tetrahydro-2-furyl)-uracil (Tegafur), 2-[Bis-(2-chlorethyl)-amino]-tetrahydro-2H-1, 3,2-oxazephosphorin-2-oxid, 1,2-Imidoprnpylsäureamid, 2-Hydroxymethyl-5-hydroxy-γ-Pyrone, 2,4,6-Trimethylpyridin, 2,4,6-Tri-2-pyridyl-1,3,5-triazin, 4-[Bis-(2-chlorethyl)-amino]-L-phenylalanin, 2-(3-Pyridyl)-piperidin, 2-2'-Bipyridin, 2-Methyl-(5-trimethyl-butyl-1-il-ol-3)-pyridin, 2-Methyl-(3-dimethyl-amino-1-propinyl)-pyridin und 2-Methyl-5-ethylen-pyridin, ist.

8. Komplex der allgemeinen Formel
D₂-M-T.
worin D Acetylaceton, M Mn und T eine Substanz, ausgewählt aus der Gruppe, bestehend aus 2,4,6-Trimethylpyridin, 2,4,6-Tri-2-pyridyl-1,3,5-triazin, 2-2'-Bipyridin, 2-(3-Pyridyl)-piperidin, 1,2-Imidopropylsäureamid und 4-[Bis-(2-chlorethyl)-amino]-L-phonylalanin, ist

## Claims

1. The use of a pharmaceutical composition comprising at least one complex of the general formula
D₂ - M - T
where
D is a β-diketone,
M is a metal atom selected from the group consisting of Cr, Cu, Mn, Fe, Ni, Co, Zn and Mo,
T is a substance having at least one N-, O- or S-containing group, and
where M participates in an electron donor-acceptor interaction with T, and M in the complex has a free coordination site, for producing an antitumor composition.

2. The use of a pharmaceutical composition as claimed in claim 1, **characterized in that** D is selected from acetylacetone and dibenzoylmethane.

3. The use of a pharmaceutical composition as claimed in claim 1 or 2, **characterized in that** T is a substance having at least one NH₂, NH, N, O or S group.

4. The use of a pharmaceutical composition as claimed in any of claims 1 to 3, **characterized in that** T is a substance selected from the group consisting of 2,4-dihydroxy-5-fluoropyrimidine, 5-fluoro-1-(tetrahydro-2-furyl)uracil(tegafur), 2-[bis(2-chloroethyl)amino]tetrahydro-2H-1,3,2-oxazephosphorine-2-oxide, 1,2-imidopropyl acid amide, 2-hydroxymethyl-5-hydroxy-γ-pyrone, 2,4,6-trimethylpyridine, 2,4,6-tri-2-pyridyl-1,3,5-triazine, 4-[bis(2-chloroethyl)amino]-L-phenylalanine, 2-(3-pyridyl)piperidine, 2-2'-bipyridine, 2-methyl-(5-trimethylbutyl-1-il-ol-3)pyridine, 2-methyl-(3-dimethylamino-1-propynyl)pyridine and 2-methyl-5-ethylenepyridine.

5. A pharmaceutical composition of the general formula
D₂ - M - T
in which D is acetylacetone, M is Cu and T is a substance selected from the group consisting of 2,4-dihydroxy-5-fluoropyrimidine, 5-fluoro-1-(tetrahydro-2-furyl)uracil, 2-[bis(2-chloroethyl)amino]tetrahydro-2H-1,3,2-oxazephosphorine-2-oxide, 1,2-imidopropyl acid amide, 2-hydroxymethyl-5-hydroxy-y-pyrone, 2,4,6-trimethylpyridine, 2,4,6-tri-2-pyridyl-1,3,5-triazine, 4-[bis(2-chloroethyl)amino]-L-phenylalanine, 2-(3-pyridyl)piperidine, 2-2'-bipyridine, 2-methyl-(5-trimethylbutyl-1-il-ol-3)pyridine, 2-methyl-(3-dimethylamino-1-propynyl)pyridine and 2-methyl-5-ethylenepyridine.

6. A pharmaceutical composition of the general formula
D₂ - M - T
in which D is acetylacetone, M is Mn and T is a substance selected from the group consisting of 2,4,6-trimethylpyridine, 2,4,6-tri-2-pyridyl-1,3,5-triazine, 2-2'-bipyridine, 2-(3-pyridyl)piperidine, 1,2-imidopropyl acid amide and 4-[bis(2-chloroethyl)amino]-L-phenylalanine.

7. Complex of the general formula
D₂ - M - T
in which D is acetylacetone, M is Cu and T is a substance selected from the group consisting of 2,4-dihydroxy-5-fluoropyrimidine, 5-fluoro-1-(tetrahydro-2-furyl)uracil(tegafur), 2-[bis(2-chloro-ethyl)-amino]tetrahydro-2H-1,3,2-oxazephosphorine-2-oxide, 1,2-imidopropyl acid amide, 2-hydroxymethyl-5-hydroxy-γ-pyrone, 2,4,6-trimethylpyridine, 2,4,6-tri-2-pyridyl-1,3,5-triazine, 4-[bis(2-chloroethyl)amino]-L-phenylalanine, 2-(3-pyridyl)piperidine, 2-2'-bipyri-dine, 2-methyl-(5-trimethylbutyl-1-il-ol-3)pyridine, 2-methyl-(3-dimethylamino-1-propynyl)pyridine and 2-methyl-5-ethylenepyridine.

8. Complex of the general formula
D₂ - M - T
in which D is acetylacetone, M is Mn and T is a substance selected from the group consisting of 2,4,6-trimethylpyridine, 2,4,6-tri-2-pyridyl-1,3,5-triazine, 2-2'-bipyridine, 2-(3-pyridyl)piperidine, 1,2-imidopropyl acid amide and 4-[bis(2-chloroethyl)amino]-L-phenylalanine.

## Revendications

1. Utilisation d'une composition pharmaceutique contenant au moins un complexe de formule générale
D₂ - M - T,
dans laquelle
D représente un β-dicétone,
M représente un atome de métal choisi dans le groupe comprenant Cr, Cu, Mn, Fe, Ni, Co, Zn et Mo,
T représente une substance comportant au moins un groupement contenant N-, O- ou S- et
moyennant quoi M entre dans une interaction donneur-accepteur d'électrons avec T et M comporte dans le complexe un site libre de coordination,
pour préparer un agent anti-tumoral.

2. Utilisation d'une composition pharmaceutique selon la revendication 1,
**caractérisée en ce que**
D est choisi parmi l'acétylacétone et le dibenzoylméthane.

3. Utilisation d'une composition pharmaceutique selon la revendication 1 ou 2,
**caractérisée en ce que**
T est une substance contenant au moins un groupement NH₂-, NH-, N-, O-ou S-.

4. Utilisation d'une composition pharmaceutique selon l'une des revendications 1 à 3,
**caractérisée en ce que**
T est une substance choisie parmi le groupe comprenant la 2,4-dihydroxy-5-fluoropyrimidine, le 5-fluoro-1-(tétrahydro-2-furyl)-uracile, le 2-[bis-(2-chloroéthyl)-amino]-tétrahydro-2H-1,3,2-oxazaphosphorin-2-oxyde, le 1,2-imidopropyl amide, le 2-hydroxyméthyl-5-hydroxy-y-pyrone, la 2,4,6-triméthylpyridine, la 2,4,6-tri-2-pyridyl-1,3,5-triazine, le 4-[bis-(2-chloroéthyl)-amino]-L-phénylalanine, la 2-(3-pyridyl)-pipéridine, la 2-2'-bipyridine, la 2-méthyl-(5-triméthyl-butyl-1-il-ol-3)-pyridine, la 2-méthyl-(3-diméthylamino-1-propinyl)-pyridine et la 2-méthyl-5-éthylène-pyridine.

5. Composition pharmaceutique de formule générale
D₂ - M - T,
dans laquelle D est de l'acétylacétone, M est Cu et T est une substance choisie parmi le groupe comprenant la 2,4-dihydroxy-5-fluoropyrimidine, le 5-fluoro-1-(tétrahydro-2-furyl)-uracile, le 2-[bis-(2-chloroéthyl)-amino]-tétrahydro-2H-1,3,2-oxazaphosphorin-2-oxyde, le 1,2-imidopropyl amide, le 2-hydroxyméthyl-5-hydroxy-y-pyrone, la 2,4,6-triméthylpyridine, la 2,4,6-tri-2-pyridyl-1,3,5-triazine, le 4-[bis-(2-chloroéthyl)-amino]-L-phénylalanine, la 2-(3-pyridyl)-pipéridine, la 2-2'-bipyridine, la 2-méthyl-(5-triméthyl-butyl-1-il-ol-3)-pyridine, la 2-méthyl-(3-diméthylamino-1-propinyl)-pyridine et la 2-méthyl-5-éthylène-pyridine.

6. Composition pharmaceutique de formule générale
D₂-M-T,
dans laquelle D est de l'acétylacétone, M est Mn et T est une substance choisie parmi le groupe comprenant la 2,4,6-triméthylpyridine, la 2,4,6-tri-2-pyridyi-1,3,5-triazine, la 2-2'-bipyridine, la 2-(3-pyridyl)-pipéridine, le 1,2-imidopropyl amide et le 4-[bis-(2-chloroéthyl)-amino]-L-phénylalanine.

7. Complexe de formule générale
D₂-M-T,
dans laquelle D est de l'acétylacétone, M est Cu et T est une substance choisie parmi le groupe comprenant la 2,4-dihydroxy-5-fluoropyrimidine, le 5-fluoro-1-(tétrahydro-2-furyl)-uracile (Tegafur), le 2-[bis-(2-chloroéthyl)-amino]-tétrahydro-2H-1,3,2-oxazaphosphorin-2-oxyde, le 1,2-imidopropyl amide, le 2-hydroxyméthyl-5-hydroxy-γ-pyrone, la 2,4,6-triméthylpyridine, la 2,4,6-tri-2-pyridyl-1,3,5-triazine, le 4-[bis-(2-chloroéthyl)-amino]-L-phénylalanine, la 2-(3-pyridyl)-pipéridine, la 2-2'-bipyridine, la 2-méthyl-(5-triméthyl-butyl-1-il-ol-3)-pyridine, la 2-méthyl-(3-diméthylamino-1-propinyl)-pyridine et la 2-méthyl-5-éthylène-pyridine.

8. Complexe de formule générale
D₂-M-T,
dans laquelle D est de l'acétylacétone, M est Mn et T est une substance choisie parmi le groupe comprenant la 2,4,6-triméthylpyridine, la 2,4,6-tri-2-pyridyl-1,3,5-triazine, la 2-2'-bipyridine, la 2-(3-pyridyl)-pipéridine, le 1,2-imidopropyl amide et le 4-[bis-(2-chloroéthyl)-amino]-L-phénylalanine.
